(19) Europäisches Patentamt
European Patent Office
Office européen des brevets

(11) Publication number: **0 320 793 B1**

(12) # EUROPEAN PATENT SPECIFICATION

(45) Date of publication of patent specification: **12.05.93**

(51) Int. Cl.[5]: **C07D 237/28**, A01N 43/58

(21) Application number: **88120512.4**

(22) Date of filing: **08.12.88**

(54) **Cinnoline derivative, process for preparing the same and herbicidal composition containing the same.**

(30) Priority: **17.12.87 JP 320276/87**

(43) Date of publication of application:
**21.06.89 Bulletin 89/25**

(45) Publication of the grant of the patent:
**12.05.93 Bulletin 93/19**

(84) Designated Contracting States:
**CH DE ES FR GB IT LI NL**

(56) References cited:
**EP-A- 0 197 226**
**EP-A- 0 320 782**

(73) Proprietor: **SUMITOMO CHEMICAL COMPANY, LIMITED**
**Kitahama 4-chome 5-33**
**Chuo-ku Osaka 541(JP)**

(72) Inventor: **Mizutani, Masato**
**2-10-5-301, Sonehigashi-machi**
**Toyonaka-shi**
**Osaka-fu(JP)**
Inventor: **Shiroshita, Masao**
**2-3-12, Miyahara Yodogawa-ku**
**Osaka-shi Osaka-fu(JP)**
Inventor: **Sakaki, Masaharu**
**2-10-3-329, Sonehigashi-machi**
**Toyonaka-shi**
**Osaka-fu(JP)**
Inventor: **Mito, Nobuaki**
**2-14-7, Mefu Takarazuka-shi**
**Hyogo-ken(JP)**
Inventor: **Okuda, Hiroki**
**1-3-7-601, Shimae-cho Toyonaka-shi**
**Osaka-fu(JP)**

(74) Representative: **Türk, Gille, Hrabal, Leifert**
**Brucknerstrasse 20**
**W-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to novel cinnoline derivatives, processes for preparing the cinnoline derivatives and herbicidal compositions containing the cinnoline derivatives as an active ingredient. The present invention further relates to a method for controlling undesired weeds using the cinnoline derivatives and use of the cinnoline derivatives as a herbicide.

Some 1-aryl-1,4-dihydro-4-oxocinnoline-3-carboxylic acid derivatives have been hitherto reported in literatures such as Zh. Obshch. Khim., vol.37, p.2487 (1967), J. Chem. Soc. Chem., Comm., p.752 (1974), Synthesis, p.52 (1983), U.S. Patent No. 4,604,134 and European Patent Publication No. 197226 A1. However, there has not been reported that cinnoline derivatives in the present invention have herbicidal activity.

As a result of the eager study for providing a novel herbicide, it has now been found that cinnoline derivatives having the formula (I):

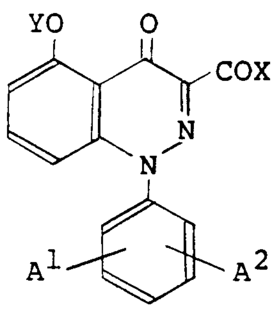

(I)

in which X is $-OH$, $-O^{-}M^{+}$, $-OR^1$ or

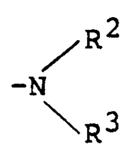

wherein $M^+$ is an alkali metal cation, an alkaline earth metal cation or

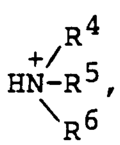

in which $R^4$, $R^5$ and $R^6$ are the same or different and each is a hydrogen atom, a $C_1-C_6$ alkyl group, a $C_3-C_4$ alkenyl group, a $C_3-C_4$ alkynyl group, a $C_3-C_8$ cycloalkyl group, a benzyl group or a phenyl group; $R^1$ is a $C_1-C_9$ alkyl group, a $C_3-C_6$ alkenyl group, a $C_3-C_4$ alkynyl group, a $C_1-C_3$ alkoxy-($C_1-C_4$) alkyl group, a $C_1-C_3$ haloalkyl group, a $C_3-C_8$ cycloalkyl group, a benzyl group or a phenyl group; and $R^2$ and $R^3$ are the same or different and each is a hydrogen atom, a $C_1-C_6$ alkyl group, a $C_3-C_4$ alkenyl group, a $C_3-C_4$ alkynyl group, a $C_3-C_8$ cycloalkyl group, a benzyl group in which at most two of hydrogen atoms at the $\alpha$-position thereof may be substituted by methyl group, a $C_2-C_3$ hydroxyalkyl group or a phenyl group in which at most three of hydrogen atoms thereof may be substituted by the same or different $C_1-C_2$ alkyl group or halogen atom;
Y is a $C_1-C_4$ haloalkyl group; $A^1$ and $A^2$ are the same or different and each is a hydrogen atom, a fluorine atom, a chlorine atom, a bromine atom, a trihalomethyl group, a $C_1-C_2$ alkylthio group of a $C_1-C_2$ haloalkoxy group exhibit both excellent herbicidal activity and selectivity between crops and weeds, and thus the present invention has been accomplished.

In accordance with the present invention, there are provided a cinnoline derivative having the formula (I), a process for preparing it, a herbicidal composition containing it as an acitve ingredient, a method for controlling undesired weeds using it and use of it as a herbicide.

Among the cinnoline derivatives having the formula (I) of the present invention, cinnoline derivatives having $-OH$, $-O^-M^+$ or $-OR^1$ as X are preferable because of the highly herbicidal activity against weeds. Further, among them, cinnoline derivatives having a fluorine atom, a chlorine atom, a bromine atom, a trifluoromethyl group, a difluoromethoxy group or a trifluoromethoxy group as $A^1$ and a hydrogen atom or a fluorine atom as $A^2$ are more preferable. Moreover, among them, cinnoline derivatives having a $C_1-C_2$ polyfluoroalkyl group (especially a difluoromethyl groups, a trifluoromethyl group or a 2,2,2-trifluoroethyl group) as Y are still more preferable.

Hereinafter, processes for preparing the cinnoline derivatives of the present invention are explained.

In the compounds of the present invention, a cinnoline derivative having the formula (I-a):

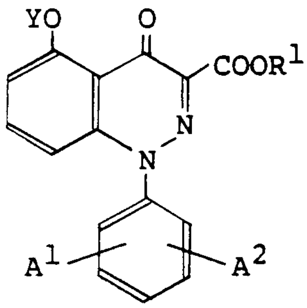

(I-a)

in which $R^1$, Y, $A^1$ and $A^2$ are as defined above can be prepared by subjecting a hydrazone having the formula (II):

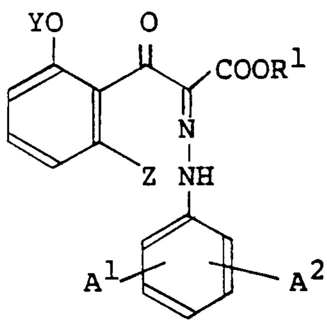

(II)

in which $R^1$, Y, $A^1$ and $A^2$ are as defined above and Z is a fluorine atom, chlorine atom or bromine atom to ring closure with a dehydrohalogenating agent.

The above reaction is usually carried out without any solvent or in a solvent, at a temperature of 0 to 150 °C, for a period of 10 minutes to 20 hours. The dehydrohalogenating agent may be used in an amount of 1 to 10 equivalents to one equivalent of the hydrazone (II).

Examples of the solvent are, for instance, aliphatic hydrocarbons (e.g. hexane, heptane, ligroin, petroleum ether), aromatic hydrocarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, diethylene glycol dimethyl ether), ketones (e.g. acetone, methyl ethyl ketone, methyl isobutyl ketone, isophorone, cyclohexanone), esters (e.g. ethyl formate, ethyl acetate, butyl acetate, diethyl carbonate), nitro compounds (e.g. nitroethane, nitrobenzene), nitriles (e.g. acetonitrile, isobutylnitrile), tertiary amines (e.g. pyridine, triethylamine, N,N-diethylaniline, tributylamine, N-methylmorpholine), acid amides (e.g. formamide, N,N-dimethylformamide, acetamide), sulfur compounds (e.g. dimethyl sulfoxide, sulfolane), water. Their mixtures are also usable.

Examples of the dehydrohalogenating agent are, for instance, organic bases (e.g. pyridine, triethyl amine, N,N-diethylaniline), inorganic bases (e.g. sodium hydroxide, potassium hydroxide, sodium carbonate, potassium carbonate, sodium hydride), alkali metal alkoxide (e.g. sodium methoxide, sodium ethoxide).

For the purpose of conducting the reaction more efficiently, quaternary ammonium salts and crown ethers can be added. Examples of the quaternary ammonium salts are, for instance, benzyltriethylammonium chloride, tetrabutylammonium chloride, and the like. Examples of the crown ethers are, for

instance, dibenzo-18-crown-6.

After completion of the reaction, the reaction mixture is subjected to ordinary post-treatment such as addition of water followed by collection of precipitated crystals, extraction with an organic solvent, or concentration. If necessary, a purification procedure such as chromatography or recrystallization may be adopted. Thus the cinnoline derivative (I-a) of the present invention can be obtained.

In the compounds of the present invention, a cinnoline derivative having the formula (I-b):

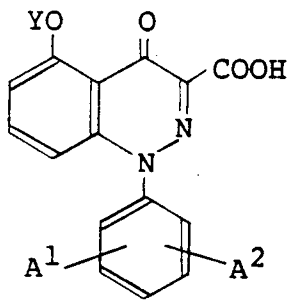

(I-b)

in which Y, $A^1$ and $A^2$ are as defined above
can be prepared by hydrolyzing the cinnoline derivative (I-a).

The above reaction is carried out in water or a mixed solvent of water and an alcohol (e.g. methanol, ethanol, isopropanol, diethylene glycol, glycerin), an ether (e.g. tetrahydrofuran, dioxane), a nitrile (e.g. acetonitrile), an acid amide (e.g. formamide, N,N-dimethylformamide) or a sulfur compound (e.g. dimethyl sulfoxide). Usually the acid or the alkali is added in an amount of 1 to 100 equivalents to one equivalent of the cinnoline derivative (I-a). The reaction temperature is 20 to 100°C. The reaction period is 30 minutes to 10 hours.

Examples of the acid are, for instance, hydrochloric acid, sulfuric acid, nitric acid, and the like. Examples of the alkali are, for instance, sodium hydroxide, potassium hydroxide, and the like. When the alkali is used, the reaction mixture is neutralized with hydrochloric acid, sulfuric acid, nitric acid, formic acid, acetic acid, or the like after completion of the reaction.

After completion of the reaction, the reaction mixture is subjected to ordinary post-treatment such as collection of precipitated crystals, extraction with an organic solvent, or concentration. If necessary, a purification procedure such as chromatography or necessary recrystallization may be adopted. Thus the cinnoline derivative (I-b) of the present invention can be obtained.

In the compounds of the present invention, a cinnoline derivative having the formula (I-c):

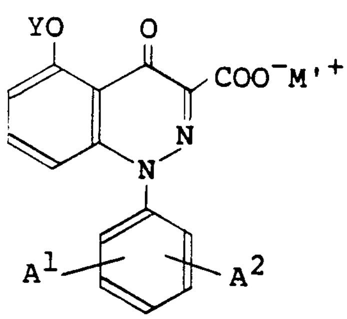

(I-c)

wherein Y, $A^1$ and $A^2$ are as defined above, and $M'^{+}$ is an alkali metal cation or an alkaline earth metal cation can be prepared by reacting the cinnoline derivative (I-b) with a hydroxide having the formula (III):

$$M'^{+}OH^{-} \qquad (III)$$

wherein $M'^{+}$ is as defined above.

The above reaction is usually carried out in water at a temperature of 0 to 50 °C for a period of 5 minutes to 5 hours. The hydroxide (III) may be used in an amount of 0.7 to 1 equivalent to one equivalent of the cinnoline derivative (I-b).

Examples of the hydroxide (III) are, for instance, lithium hydroxide, sodium hydroxide, potassium hydroxide, calcium hydroxide.

After completion of the reaction, if necessary, the water layer is washed with an organic solvent, and then concentrated. Thus the cinnoline derivative (I-c) of the present invention can be obtained.

In the compounds of the present invention, a cinnoline derivative having the formula (I-d):

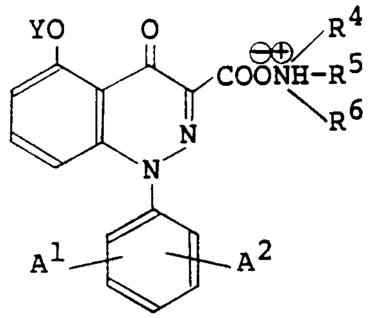

(I-d)

wherein $R^4$, $R^5$, $R^6$, Y, $A^1$ and $A^2$ are as defined above can be prepared by reacting the cinnoline derivative (I-b) with an amine having the formula (IV):

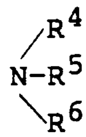

(IV)

wherein $R^4$, $R^5$ and $R^6$ are as defined above.

The above reaction is usually carried out without any solvent or in a solvent, at a temperature of 0 to 100 °C, for a period of 5 minutes to 3 hours. The amine (IV) may be used in an amount of 1 to 10 equivalents to one equivalent of the cinnioline derivative (I-b).

Examples of the solvent are, for instance, aromatic hydrocarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, diethylene glycol dimethyl ether), alcohols (e.g. methanol, ethanol, isopropanol, t-butanol, octanol, cyclohexanol, methyl cellosolve, diethylene glycol, glycerin), esters (e.g. ethyl formate, ethyl acetate, butyl acetate, diethyl carbonate), nitro compounds (e.g. nitroethane, nitrobenzene), nitriles (e.g. acetonitrile, isobutylnitrile), water. Their mixtures are also usuable.

After completion of the reaction, the reaction mixture is subjected to ordinary post-treatment such as concentration. If necessary, a purification procedure such as recrystallization may be adopted. Thus the cinnoline derivative (I-d) can be obtained.

In the compounds of the present invention, a cinnoline derivative (I-e):

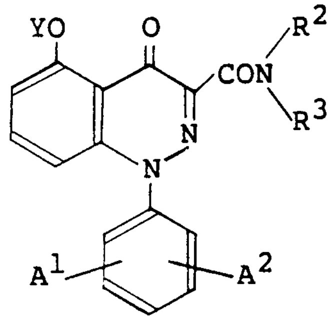

(I-e)

wherein $R^2$, $R^3$, Y, $A^1$ and $A^2$ are as defined above can be prepared by reacting a halide having the formula (V):

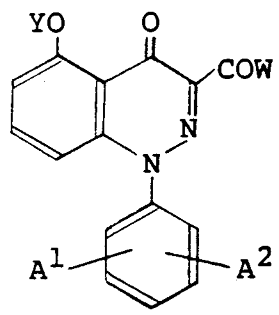

(V)

wherein Y, $A^1$ and $A^2$ are as defined above and W is a halogen atom with an amine having the formula (VI):

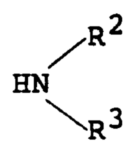

(VI)

wherein $R^2$ and $R^3$ are as defined above.

The above reaction is usually carried out without any solvent or in a solvent, in the presence of a dehydrohalogenating agent, at a temperature of 0 to 50 ° C, for a period of 10 minutes to 3 hours. The amine (VI) and the dehydrohalogenating agent are used in an amount of 1 to 5 equivalents and 1 to 2 equivalents respectively, to one equivalent of the halide (V).

Examples of the solvents are, for instance, aliphatic hydrocarbons (e.g. hexane, heptane, ligroin, petroleum ether), aromatic hydrocarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, diethylene glycol dimethyl ether), esters (e.g. ethyl formate, ethyl acetate, butyl acetate, diethyl carbonate), nitro compounds (e.g. nitroethane, nitrobenzene), nitriles (e.g. acetonitrile, isobutylnitrile), tertiary amines (e.g. pyridine, triethylamine, N,N-diethylaniline, tributylamine, N-methylmorpholine), acid amides (e.g. formamide, N,N-dimethylformamide, acetamide), sulfur compound (e.g. dimethyl sulfoxide, sulfolane), water. Their mixtures are also usable.

Examples of the dehydrohalogenating agent are, for instnace, organic bases (e.g. pyridine, triethyl amine, N,N-diethyl aniline).

After completion of the reaction, the reaction mixture is subjected to ordinary post-treatment such as extraction with an organic solvent or concentration. If necessary, a purification procedure such as chromatography or recrystallization may be adopted. Thus the cinnoline derivative (I-e) can be obtained.

The halide (V) is easily prepared by usual acid halogenation of the cinnoline derivative (I-b).

Typical examples of the cinnoline derivatives which can be prepared according to the above procedures are shown in Table 1.

Table 1

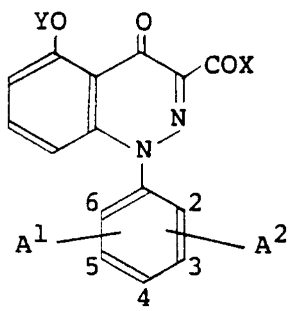

| $A^1$ | $A^2$ | X | Y |
|---|---|---|---|
| H | H | OH | $CHF_2$ |
| " | " | OK | " |
| " | " | $OC_2H_5$ | " |
| 2-Cl | " | " | " |
| 4-Cl | " | " | " |
| " | " | OH | " |
| " | " | OK | " |
| " | " | $ONH_4$ | " |
| " | " | $N(CH_3)_2$ | " |
| 2-F | 4-Cl | $OC_4H_9$-n | " |
| " | " | $OC_2H_5$ | " |
| 3-Cl | 4-F | " | " |
| 2-Cl | 4-Cl | " | " |
| 4-Br | H | OH | " |
| " | " | NH-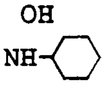 | " |
| 2-F | 4-Br | $OC_3H_7$-i | " |
| $4-CF_3$ | H | OH | " |
| " | " | ONa | " |
| " | " | $OCH_3$ | " |
| $4-SCH_3$ | " | OH | " |
| " | " | OK | " |
| $4-SCH_3$ | " | $OC_2H_5$ | " |

- continued -

- continued -

| $A^1$ | $A^2$ | X | Y |
|---|---|---|---|
| 4-$OCHF_2$ | H | $OCH_3$ | $CHF_2$ |
| 2-F | 4-$OCHF_2$ | OK | " |
| 3-$OCF_3$ | H | $OC_2H_5$ | " |
| 4-$OCF_3$ | " | " | " |
| " | " | OH | " |
| " | " | ONa | " |
| " | " | O($\frac{1}{2}$Ca) | " |
| " | " | $ONH(C_2H_5)_3$ | " |
| 4-$OCF_2Br$ | " | $OC_2H_5$ | " |
| 4-$OCF_2CHCl_2$ | " | " | " |
| H | " | OH | $CF_3$ |
| " | " | ONa | " |
| " | " | $OC_2H_5$ | " |
| 4-F | " | " | " |
| 4-Cl | " | OH | " |
| " | " | OK | " |
| " | " | $OC_2H_5$ | " |
| " | " | 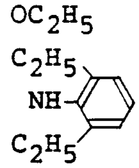 | " |
| 3-F | 4-Cl | OH | " |
| " | " | OK | " |
| " | " | $OCH_3$ | " |
| " | " | $OC_4H_9$-n | " |
| 2-Cl | " | $OC_2H_5$ | " |
| 4-Br | H | $OCH_3$ | " |
| 2-F | 4-Br | $OC_2H_5$ | " |
| 3-$CF_3$ | H | $OCH_3$ | " |
| 4-$CF_3$ | " | $OC_3H_7$-i | " |
| 4-$SCH_3$ | " | OH | " |
| " | " | ONa | " |
| " | " | $OC_2H_5$ | " |

- continued -

- continued -

| $A^1$ | $A^2$ | X | Y |
|---|---|---|---|
| 4-$OCHF_2$ | H | $OC_2H_5$ | $CF_3$ |
| 2-$OCHF_2$ | 4-F | $OCH_3$ | " |
| 4-$OCF_3$ | H | OH | " |
| " | " | OK | " |
| " | " | $OC_2H_5$ | " |
| 4-$OCF_2Br$ | " | OK | " |
| H | " | $OC_2H_5$ | $CF_2Br$ |
| 4-Cl | " | OK | " |
| 2-F | 4-Cl | $OC_3H_7$-i | " |
| 4-$SCH_3$ | H | $OC_3H_7$-n | " |
| 4-$OCHF_2$ | " | OH | " |
| 4-$OCF_3$ | " | $OC_2H_5$ | " |
| H | " | OH | $CF_2CF_2H$ |
| " | " | ONa | " |
| " | " | $OC_2H_5$ | " |
| 2-Cl | " | $OCH_3$ | " |
| 4-Cl | " | $OC_2H_5$ | " |
| 2-F | 4-Cl | $OCH_3$ | " |
| 3-F | " | $OC_2H_5$ | " |
| 3-Cl | 4-F | OK | " |
| 4-Br | H | OH | " |
| 4-$CF_3$ | " | " | " |
| 4-$SCH_3$ | " | OK | " |
| " | " | $OCH_3$ | " |
| 4-$OCF_3$ | " | $OC_2H_5$ | " |
| 4-$CF_2Br$ | " | OH | " |
| 4-F | " | $OC_2H_5$ | $CH_2CF_3$ |
| 4-Cl | " | " | " |
| 4-Br | " | OH | " |
| 4-$CF_3$ | " | OK | " |
| 4-$SCH_3$ | " | OH | " |
| 2-F | 4-$OCHF_2$ | $OC_2H_5$ | " |

- continued -

- continued -

| $A^1$ | $A^2$ | X | Y |
|---|---|---|---|
| 2-Cl | 4-F | $OC_3H_7$-n | $CF_2CHCl_2$ |
| 3-Cl | " | $OCH_3$ | " |
| 4-$SCH_3$ | H | $OC_2H_5$ | " |
| 4-$OCF_3$ | " | OK | " |
| H | " | $OC_2H_5$ | $CF_2CHFCl$ |
| 4-F | " | " | " |
| " | " | OH | " |
| " | " | OK | " |
| 4-Br | " | $OCH_3$ | " |
| 2-F | 4-Br | OH | " |
| 4-$OCF_3$ | H | $OC_2H_5$ | " |
| 4-Cl | " | OK | CFClCHFCl |
| 3-Cl | 4-Cl | $OC_2H_5$ | " |
| 4-$CF_3$ | H | $OC_3H_7$-n | " |
| 4-$SCH_3$ | " | OH | " |
| 4-$OCHF_2$ | " | " | " |
| H | " | " | $CF_2CHFCF_3$ |
| 4-F | " | $OC_2H_5$ | " |
| 4-Cl | " | OK | " |
| 2-F | 4-Cl | $OCH_3$ | " |
| 4-$CF_3$ | H | $OC_2H_5$ | " |
| 4-$SC_2H_5$ | " | OH | " |
| 4-$OCF_3$ | " | ONa | " |

In preparing the compound of the present invention, the hydrazone derivative (II), which is the starting material, can be prepared by reacting a ketoester having the formula (VII):

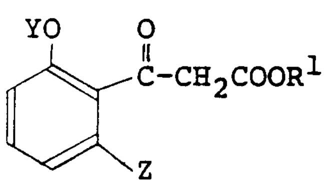

(VII)

in which $R^1$, Y and Z are as defined above with a diazonium salt having the formula (VIII):

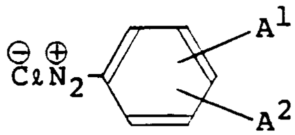

(VIII)

in which $A^1$ and $A^2$ are as defined above.

The above reaction is usually carried out in a solvent at a temperature of 0 to 50 °C for a period of 10 minutes to 5 hours. The diazonium salt (VIII) may be used in an amount of 0.7 to 1.5 equivalents to one equivalent of ketoester (VII).

Examples of the solvent are, for instance, ethers (e.g. diethyl ether, diisopropyl ether, dioxane, tetrahydrofuran, diethylene glycol dimethyl ether), alcohols (e.g. methanol, ethanol, isopropanol, t-butanol, octanol, cyclohexanol, methyl cellosolve, diethylene glycol, glycerin), tertiary amines (e.g. pyridine, triethylamine, N,N-diethylaniline, tributylamine, N-methylmorpholine), acid amides (e.g. formamide, N,N-dimethylformamide, acetamide), water. Their mixture are also usable.

For the purpose of conducting the reaction more efficiently, inorganic bases (e.g. sodium carbonate, potassium carbonate, sodium acetate, potassium acetate) can be added to the reaction system.

After completion of the reaction, the reaction mixture is subjected to ordinary post-treatment such as extraction with an organic solvent or concentration. If necessary, a purification procedure such as chromatography or recrystallization may be adopted. Thus the desired starting material (II) can be obtained.

The diazonium salt (VIII) is prepared according to an ordinary process from an aniline having the formula (IX):

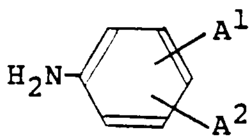

(IX)

in which $A^1$ and $A^2$ are as defined above.

Moreover, the ketoester having the formula (VII) can be prepared in accordance with the following steps.

[Step 1]

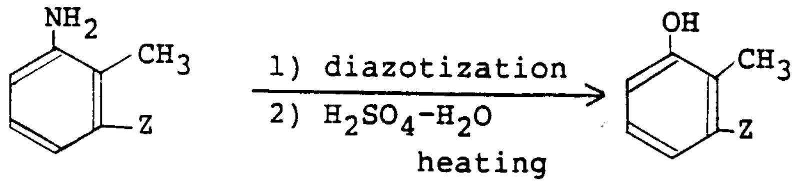


[Step 2]

Case (i) Y : $CF_3$

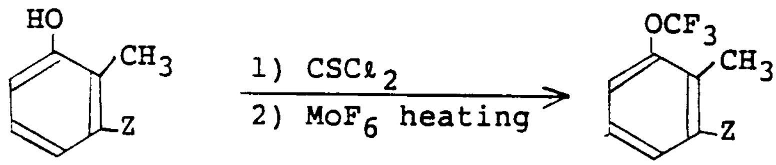


Case (ii) Y : $CH_2CF_3$

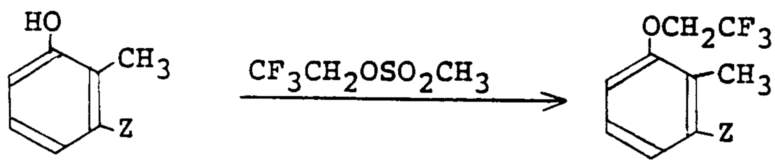


Case (iii) Y: other than Cases (i) and (ii)

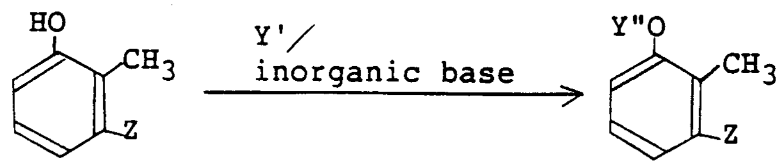


[Step 3]

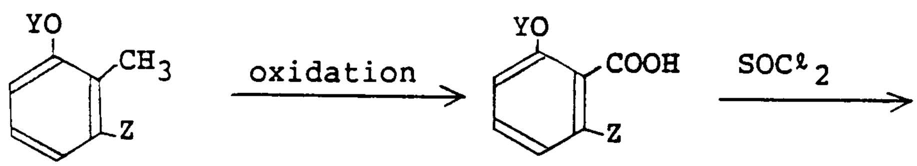


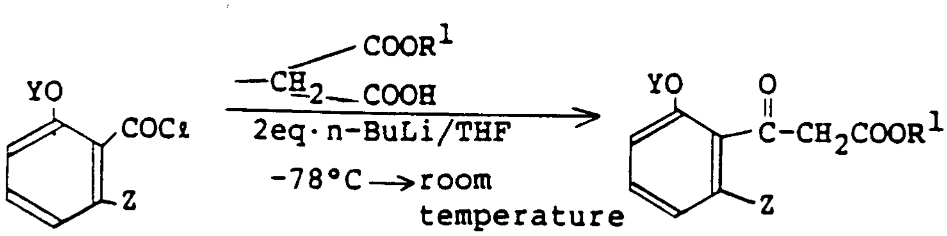


(VII)

In the above schema, $R^1$, Y and Z are as defined above, Y' is a polyhalogenated hydrocarbon, which is capable of being added to a phenol and Y" is a $C_1-C_4$ haloalkyl group except $-CF_3$ and $-CH_2CF_3$.

The compounds of the present invention show an excellent herbicidal activity and an excellent selectivity between crops and weeds. That is, in upland fields, by soil treatment or foliage treatment, the cinnoline derivatives of the present invention exhibit a herbicidal activity against undesired weeds, for instance, broad-leaved weeds such as wild buckwheat (Polygonum convolvulus), pale smartweed (Polygonum lapathifolium), common purslane (Portulaca oleracea), common chickweed (Stellaria media), common lambsquarters (Chenopodium album), redroot pigweed (Amaranthus retroflexus), radish (Raphanus sativus), wild mustard (Sinapis arvensis), shepherdspurse (Capsella bursapastoris), velvetleaf (Abutilon theophrasti), prickly sida (Sida spinosa), field pansy (Viola arvensis), cleavers (Galium aparine), field bindweed (Convolvulus arvensis), purple deadnettle (Lamium purpureum), henbit (Lamium amplexicaure), jimsonweed (Datura stramonium), black nightshade (Solanum nigrum), persian speedwell (Veronica persica), scentless chamomile (Matricaria perforata) and corn marigold (Chrysanthemum segetum); graminaceous weeds such as Japanese millet (Echinochloa frumentacea), barnyardgrass (Echinochloa crus-galli), green foxtail (Setaria virides), large crabgrass (Digitaria sanguinalis), annual bluegrass (Poa annua), blackgrass (Alopecurus myosuroides), oats (Avena sativa), wild oat (Avena fatua), johnsongrass (Sorghum halepense), quackgrass (Agropyron repens), downy brome (Bromus tectorum) and bermudagrass (Cynodon dactylon); commelinaceous weeds such as asiatic dayflower (Commelina communis); cyperaceous weeds such as purple nutsedge (Cyperus rotundus) and rice flatsedge (Cyperus iria); and the like. The cinnoline derivatives of the present invention do not exert any material phytotoxicity to main crops such as corn, wheat, rice, soybean, cotton and sugar beat.

The cinnoline derivatives of the present invention also exhibit a herbicidal activity on various lowland weeds in question, for instance, graminaceous weeds such as barnyardgrass (Echinochloa oryzicola); broad-leaved weeds such as common falsepimpernel (Lindernia procumbens), indian toothcup (Rotala indica) and waterwort (Elatine triandra); cyperaceous weeds such as smallflower umbrellaplant (Cyperus difformis), hardstem bulrush (Scirpus juncoides), needle spikerush (Eleocharis acicularis) and water nutsedge (Cyperus serotinus); and the like without exerting any material phytotoxicity to rice plants in treatment under flooded condition.

When the cinnoline derivatives are employed as an active ingredient in a herbicidal composition of the present invention, they are usually formulated in the form of emulsifiable concentrates, wettable powders, suspensions, granules in combination with auxiliary agents such as a solid carrier, liquid carrier and surface active agent. The content of the cinnoline derivatives of the present invention as the active ingredient in such formulations is within a range of 0.1 to 90 % by weight, preferably 0.2 to 80 % by weight.

Examples of the solid carrier are, for instance, fine powders or granules of kaolin clay, attapulgite clay, bentonite, terra alba, pyrophyllite, talc, diatomaceous earth, calcite, walnut powders, urea, ammonium sulfate, synthetic hydrous silicate, and the like. Examples of the liquid carrier are, for instance, aromatic hydrocarbons (e.g. xylene, methylnaphthalene), alcohols (e.g. isopropanol, ethylene glycol, cellosolve), ketones (e.g. acetone, cyclohexanone, isophorone), vegetable oils (e.g. soybean oil, cotton seed oil), dimethylsulfoxide, N,N-dimethylformamide, acetonitrile, water.

Examples of the surface active agent used for emulsification, dispersion or wetting are, for instance, anionic surface active agents such as alkylsulfates, alkylsulfonates, alkylarylsulfonates, dialkylsulfosuccinates and polyoxyethylenealkylaryl ether phosphates; non-ionic surface active agents such as polyoxyethylene alkyl ethers, polyoxyethylene alkylaryl ethers, polyoxyethylene polyoxypropylene block copolymer, sorbitan fatty acid esters and polyoxyethylene sorbitan fatty acid esters.

Examples of the auxiliary agents other than above are, for instance, ligninsulfonates, alginates, polyvinyl alcohols, gum arabic, CMC (carboxymethyl cellulose), PAP (isopropyl acid phosphate).

As the method for controlling undesired weeds of the present invention, the cinnoline derivatives of the present invention are usually formulated and used in soil treatment, foliage treatment or treatment under flooded condition before the emergence of weeds or within about one month after the emergence of weeds to the area where undesired weeds grow or will grow. Soil treatment includes soil surface treatment, soil incorporation treatment. The foliage treatment includes, in addition to the treatment of the plant over the top, directed application wherein herbicides are applied only to weeds so as not to attach to crops.

The cinnoline derivatives of the present invention may be used together with other herbicides to improve their activity as herbicides.

Further, they may applied in combination with insecticides, acaricides, nematocides, fungicides, plant growth regulators, fertilizers, soil improvers.

Furthermore, the cinnoline derivatives of the present invention can be used as an active ingredient of a herbicide for paddy field, upland field, orchard, pasture land, lawn, forest, non-agricultural field.

The dosage rate of the cinnoline derivatives of the present invention varies depending on weather conditions, preparation form, prevailing season, mode of application, soil involved, crop and weed species aimed at. Generally, however, the dosage rate is from 0.5 to 500 grams, preferably from 1 to 300 grams of the active ingredient per are. The herbicidal composition of the invention formulated into emulsifiable concentrate, a wettable powder, a suspension, or the like is ordinarily employed by diluting a designed amount of it with water, if necessary with addition of an auxiliary agent such as a spreading agent, at a

volume of 1 to 10 liters per are. The herbicidal composition formulated into granule is usually applied without any dilution.

Examples of the wetting agent are, in addition to the surface active agents as noted above, for instance, polyoxyethylene resin acid (ester), ligninsulfonate, abiethylenic acid salt, dinaphthylmethanedisulfonate, paraffin.

The present invention is more specifically described and explained by means of the following Examples, Reference Example, Formulation Examples and Test Examples, wherein the compound Nos. of the active ingredient corresponds to those in Table 2.

Example 1

[Preparation of the compound No. 33]

Ethyl 2-[(4-trifluoromethoxyphenyl)-1,1-diazanediyl]-(2-fluoro-6-difluoromethoxybenzoyl)-acetate (530 mg), and potassium carbonate (157 mg) were added to N,N-dimethylformamide (10 mℓ), and the resultant mixture was heated at 100°C for 1 hour. The mixture was cooled to room temperature, and was poured into ice-water (about 50 mℓ). After allowing it to stand for 2 hours, the precipitated crystals were collected by filtration. The crystals were washed with water (5 mℓ) two times and were dried under reduced pressure to give 478 mg of desired ethyl 1-(4-trifluoromethoxyphenyl)-1,4-dihydro-4-oxo-5-difluoromethoxycinnoline-3-carboxylate (yield: 94.3 %). m.p., 145°C

Example 2

[Preparation of the compound No. 15]

Ethyl 2-[(2-fluoro-4-chlorophenyl)-1,1-diazanediyl]-(2-fluoro-6-difluoromethoxybenzoyl)-acetate (460 mg) and potassium carbonate (137 mg) were added to N,N-dimethylformamide (10 mℓ), and the resultant mixture was heated at 100°C for 1 hour. The mixture was cooled to room temperature, and was poured into ice-water (about 50 mℓ). After allowing it to stand overnight, the precipitated crystals were collected by filtration, washed with water (5 mℓ) two times and were dried under reduced pressure to give 410 mg of desired ethyl 1-(2-fluoro-4-chlorophenyl)-1,4-dihydro-4-oxo-5-difluoromethoxycinnoline-3-carboxylate (yield: 93.8 %). m.p., 140-142°C

Example 3

[Preparation of the compound No. 30]

Ethyl 1-(4-trifluoromethoxyphenyl)-1,4-dihydro-4-oxo-5-difluoromethoxycinnoline-3-carboxylate (200 mg) and a 1 N aqueous solution of sodium hydroxide (0.93 mℓ) were added to a mixed solvent of ethanol (5.5 mℓ) and water (1.5 mℓ), and the resultant mixture was stirred at 70 to 80°C for 3 hours. After being allowed to cool to room temperature, the mixture was diluted with water (50 mℓ), and washed with ethyl acetate (20 mℓ). The pH of the water layer was adjusted to pH 2 with concentrated hydrochloric acid to precipitate crystals. The precipitated crystals were collected by filtration, washed with water (5 mℓ) two times, and dried under reduced pressure to give 120 mg of desired 1-(4-trifluoromethoxyphenyl)-1,4-dihydro-4-oxo-5-difluoromethoxycinnoline-3-carboxylic acid (yield: 64.2 %). m.p., 236.5°C

Example 4

[Preparation of the compound No. 31]

1-(4-Trifluoromethoxyphenyl)-1,4-dihydro-4-oxo-5-difluoromethoxycinnoline-3-carboxylic acid (120 mg) and a 1 N aqueous solution of sodium hydroxide (0.265 mℓ) were added to water (5 mℓ), and the mixture was stirred at room temperature for 1 hour. The resultant mixture was washed with ethyl acetate (10 mℓ). After removing the water, the obtained crystals were dried to give 91 mg of desired sodium 1-(4-trifluoromethoxyphenyl)-1,4-dihydro-4-oxo-5-difluoromethoxycinnoline-3-carboxylate (yield: 78.4 %). m.p., 165-169°C

Some of the cinnoline derivatives of the present invention obtained according to these process as

above are shown in Table 2.

Table 2

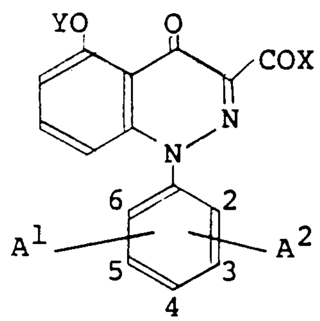

(I)

| Compound No. | $A^1$ | $A^2$ | X | Y | Melting point (°C) |
|---|---|---|---|---|---|
| 1 | H | H | OH | $CHF_2$ | 261-263 |
| 2 | " | " | OK | " | 277-279 |
| 3 | " | " | $OC_2H_5$ | " | 159-161 |
| 4 | 4-F | " | ONa | " | 248-255 |
| 5 | " | " | $OC_2H_5$ | " | 185.5 |
| 6 | 2-Cℓ | " | " | " | 191.7 |
| 7 | 4-Cℓ | " | OH | " | 256.8 |
| 8 | " | " | OK | " | 270-275 |
| 9 | " | " | $OC_2H_5$ | " | 198.3 |
| 10 | 4-Br | " | OH | " | 260-263 |
| 11 | " | " | ONa | " | 211-215 |
| 12 | " | " | $OC_2H_5$ | " | 192-194 |
| 13 | 2-F | 4-Cℓ | OH | " | 206.5 |
| 14 | " | " | ONa | " | 240-246 |
| 15 | " | " | $OC_2H_5$ | " | 140-142 |
| 16 | " | 4-Br | OH | " | 208-210 |
| 17 | " | " | $OC_2H_5$ | " | 137-140 |
| 18 | 2-Cℓ | 4-F | " | " | 142-146 |
| 19 | " | 4-Cℓ | " | " | 176 |
| 20 | 3-Cℓ | 4-F | OH | " | 266-268 |
| 21 | " | " | ONa | " | 260-265 |
| 22 | " | " | $OC_2H_5$ | " | 138-144 |
| 23 | $4-CF_3$ | H | OH | " | 260-263 |
| 24 | " | " | ONa | " | 240-250 |
| 25 | " | " | OK | " | >300 |
| 26 | " | " | $OC_2H_5$ | " | 192.8 |
| 27 | $4-OCHF_2$ | " | OH | " | 237-241 |
| 28 | " | " | ONa | " | 183-187 |
| 29 | " | " | $OC_2H_5$ | " | 141.7 |
| 30 | $4-OCF_3$ | " | OH | " | 236.5 |
| 31 | " | " | ONa | " | 165-169 |

- continued -

- continued -

| Compound No. | $A^1$ | $A^2$ | X | Y | Melting point (°C) |
|---|---|---|---|---|---|
| 32 | 4-$OCF_3$ | H | OK | $CHF_2$ | 267-270 |
| 33 | " | " | $OC_2H_5$ | " | 145 |
| 34 | 4-$SCH_3$ | " | OH | " | 253-256 |
| 35 | " | " | $OC_2H_5$ | " | 152-156 |
| 36 | 4-$OCF_2CHF_2$ | " | " | " | 162.1 |
| 37 | 4-Cℓ | " | OH | $CF_3$ | 264-268 |
| 38 | " | " | OK | " | 270-274 |
| 39 | " | " | $OC_2H_5$ | " | 207-209 |
| 40 | 4-$OCF_3$ | " | OH | " | 269-270 |
| 41 | " | " | OK | " | 205-210 |
| 42 | " | " | $OC_2H_5$ | " | 145-148 |
| 43 | 4-Cℓ | " | " | $CH_2CF_3$ | 172.9 |
| 44 | 4-Br | " | " | " | 176.6 |
| 45 | 4-Cℓ | " | OH | $CF_2CHF_2$ | 197-200 |
| 46 | " | " | OK | " | 265-272 |
| 47 | " | " | $OC_2H_5$ | " | 127-130 |
| 48 | 4-$OCF_3$ | " | OH | " | 254-261 |
| 49 | " | " | OK | " | 290-300 |
| 50 | " | " | $OC_2H_5$ | " | 147-149 |

An example of process for preparing the hydrazone derivative (II), which is the starting material of the compound of the present invention is shown in the following Reference Example.

Reference Example 1

To 4-trifluoromethoxyaniline (289 mg) were added water (3 mℓ) and concentrated hydrochloric acid (1 mℓ) to give a solution. To the solution was added dropwise a solution of sodium nitrite (124 mg) in water (2 mℓ) over about 5 minutes while cooling with ice to give a solution of a diazonium salt. The obtained solution was added dropwise to a solution of ethyl 2-fluoro-6-difluoromethoxybenzoyl acetate (500 mg, purity 90 %) in a mixed solvent of 70 % methanol (8 mℓ) and pridine (0.8 mℓ) at 10 to 20 °C over about 10 minutes. After the reaction mixture was stirred at room temperature for 1 hour, the resultant mixture was added to ice-water (30 mℓ) containing a 1N hydrochloric acid (2 mℓ), the resultant mixture was extracted with ethyl acetate (30 mℓ) two times. The extract was washed with water (20 mℓ) and a saturated saline solution (20 mℓ) successively, and was dried over anhydrous magnesium sulfate. The solid obtained by removing ethyl acetate under reduced pressure was washed with a mixed solvent of hexane and ethanol to give 530 mg of desired ethyl 2-[(4-trifluoromethoxyphenyl)-1,1-diazanediyl]-(2-fluoro-6-difluoromethoxybenzoyl)-acetate (yield: 70 %). m.p., 80.5 °C

Formulation Examples are shown below. In the Formulation Examples, all parts are by weight.

Formulation Example 1

Fifty parts of any one of Compound Nos. 5, 6, 7, 8, 9, 12, 15, 19, 26, 27, 29, 32, 33, 43 and 44, 3 parts of calcium ligninsulfonate, 2 parts of sodium laurylsulfate and 45 parts of synthetic hydrous silicate are well mixed to obtain a wettable powder.

Formulation Example 2

Ten parts of any one of Compound Nos. 5, 6, 7, 9, 12, 15, 19, 26, 27, 29, 33, 43 and 44, 14 parts of polyoxyethylenestyrylphenyl ether, 6 parts of calcium dodecylbenzenesulfonate, 30 parts of xylene and 40 parts of cyclohexanone are well mixed to obtain an emulsifiable concentrate.

Formulation Example 3

Two parts of any one of Compound Nos. 5, 6, 8, 9, 12, 15, 26, 29, 32 and 43, 1 part of synthetic hydrous silicate, 2 parts of calcium ligninsulfonate, 30 parts of bentonite and 65 parts of kaolin clay are well pulverized and mixed together. The mixture is then kneaded with water, granulated and dried to obtain a granule.

Formulation Example 4

Twenty-five parts of any one of Compound Nos. 5, 6, 7, 8, 9, 12, 15, 19, 26, 27, 29, 32, 33, 43 and 44, 3 parts of polyoxyethylene sorbitan monooleate, 3 parts of CMC and 69 parts of water are mixed and pulverized until the particle size of the mixture becomes not more than 5 microns to obtain a suspension.

Formulation Example 5

Three parts of any one of Compound Nos. 2, 8, 25, 32, 38 and 41, 1 part of polyoxyethylenestyrylphenyl ether and 96 parts of water are well mixed to obtain a liquid formulation.

The herbicidal activity of the compounds are illustratively shown in the following Test Examples wherein the degree of germination and the degree of growth inhibition are observed with the naked eye and herbicidal activity is rated with an index 0, 1, 2, 3, 4, 5, 6, 7, 8, 9 or 10, in which the numeral "0" indicates no material difference is seen in comparison with the untreated plant and the numeral "10" indicates the complete inhibition or death of the test plants. Compounds A, B, C, D and E used for comparison are shown in Table 3.

Table 3

| Compound No. | Formula | Remarks |
| --- | --- | --- |
| A | 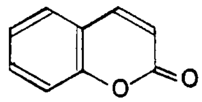 | coumarin |
| B | 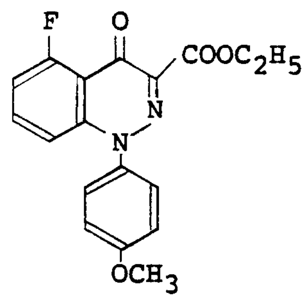 | the compound disclosed in European Patent Publication No. 197226 A1 |
| C | 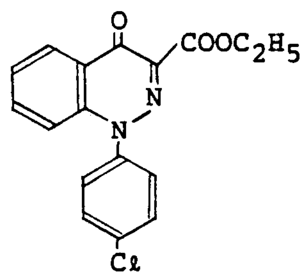 | " |
| D | 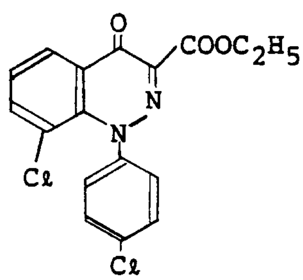 | " |
| E | 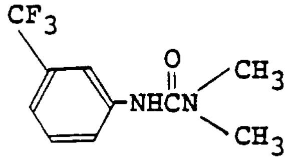 | fluometuron |

Test Example 1

Plow-field soil was filled in a cylindrical plastic pot (diameter: 10 cm, height: 10 cm), and the seeds of Japanese millet and oats were sowed and covered with the soil. A prescribed amount of the test compound

formulated into emulsifiable concentrate or liquid formulation according to Formulation Example 2 or 5 was diluted with water of an amount corresponding to 10 liters/are, and applied to the soil surface by means of a small-sized sprayer. After treatment, the test plants were cultivated for 20 days in a greenhouse to examine the herbicidal activity. The results are shown in Table 4.

Table 4

| Compound No. | Dosage (g/are) | Herbicidal activity | |
|---|---|---|---|
| | | Japanese millet | Oats |
| 5 | 40 | 10 | 10 |
| 6 | 40 | 10 | 10 |
| 8 | 40 | 10 | 10 |
| 9 | 40 | 10 | 10 |
| 12 | 40 | 10 | 10 |
| 15 | 40 | 10 | 10 |
| 19 | 40 | 10 | 10 |
| 26 | 40 | 10 | 10 |
| 27 | 40 | 10 | 10 |
| 29 | 40 | 10 | 10 |
| 33 | 40 | 10 | 10 |
| 43 | 40 | 10 | 10 |
| 44 | 40 | 10 | 10 |

Test Example 2

The seeds of Japanese millet, oats and radish were sowed in cylindrical plastic pots (diameter: 10 cm, height: 10 cm) filled with plow-field soil, and cultivated in a greenhouse for 10 days. A prescribed amount of the test compound formulated into emulsifiable concentrate or liquid formulation according to Formulation Example 2 or 5 was diluted with water containing a spreading agent, and the dilution was sprayed over the foliage of the test plants by means of a small-sized sprayer at a spray volume of 10 liters/are. The test plants were further grown in a greenhouse for 20 days, and the herbicidal activity was rated. The results are shown in Table 5.

Table 5

| Compound No. | Dosage (g/are) | Herbicidal activity | | |
|---|---|---|---|---|
| | | Japanese millet | Oats | Radish |
| 5 | 40<br>10 | 9<br>7 | 9<br>9 | 10<br>10 |
| 6 | 40<br>10 | 9<br>– | 9<br>– | 9<br>9 |
| 7 | 40<br>10 | 10<br>10 | 10<br>9 | 10<br>10 |
| 8 | 40<br>10 | 10<br>10 | 10<br>10 | 10<br>10 |
| 9 | 40<br>10 | 9<br>7 | 9<br>8 | 9<br>7 |
| 12 | 40<br>10 | 9<br>8 | 9<br>8 | 9<br>8 |
| 15 | 40<br>10 | 9<br>7 | 9<br>8 | 9<br>8 |
| 19 | 40<br>10 | 9<br>– | 8<br>– | 9<br>8 |
| 26 | 40<br>10 | 9<br>– | 9<br>7 | 10<br>10 |
| 27 | 40<br>10 | 10<br>7 | 9<br>9 | 9<br>8 |
| 29 | 40<br>10 | 9<br>– | 8<br>7 | 9<br>7 |
| 32 | 40<br>10 | 9<br>8 | 9<br>8 | 9<br>8 |
| 33 | 40<br>10 | 9<br>9 | 8<br>7 | 10<br>10 |
| 43 | 40<br>10 | 9<br>8 | 9<br>7 | 9<br>– |
| 44 | 40<br>10 | 9<br>8 | 9<br>8 | 9<br>– |

Test Example 3

The seeds of velvetleaf were sowed in cylindrical plastic pots (diameter: 10 cm, height: 10 cm) filled with plow-field soil, and cultivated in a greenhouse for 10 days. A prescribed amount of the test compound formulated into emulsifiable concentrate or liquid formulation according to Formulation Example 2 or 5 was diluted with water containing a spreading agent, and the dilution was sprayed over the foliage of the test plants by means of a small-sized sprayer at a spray volume of 10 liters/are. The test plants were further grown in a greenhouse for 20 days, and the herbicidal activity was rated. The results are shown in Table 6.

Table 6

| Compound No. | Dosage (g/are) | Herbicidal activity |
|---|---|---|
| | | Velvetleaf |
| 5 | 40 | 10 |
| | 10 | 7 |
| 6 | 40 | 7 |
| 7 | 40 | 7 |
| 9 | 40 | 7 |
| 26 | 40 | 7 |

Test Example 4

Cylindrical plastic pots (diameter: 8 cm, height: 12 cm) were filled with paddy field soil, and the seeds of barnyardgrass were sowed in 1 to 2 cm depth. Water was poured therein to make a flooded condition. The test plants were grown in a greenhouse. Six days (at that time weeds began to germinate) thereafter, a prescribed amount of the test compound formulated into emulsifiable concentrate or liquid formulation according to Formulation Example 2 or 5 was diluted with water (5 mℓ) and applied to the water surface. The test plants were grown for further 20 days in the greenhouse, and the herbicidal activity was rated. The results are shown in Table 7.

Table 7

| Compound No. | Dosage (g/are) | Herbicidal activity |
|---|---|---|
| | | Barnyardgrass |
| 1 | 40 | 10 |
| 2 | 40 | 10 |
| 3 | 40 | 10 |
| 5 | 40 | 10 |
| 6 | 40 | 10 |
| 7 | 40 | 10 |
| 8 | 40 | 10 |
| 9 | 40 | 10 |
| 10 | 40 | 10 |
| 12 | 40 | 10 |
| 15 | 40 | 10 |
| 19 | 40 | 10 |
| 23 | 40 | 10 |
| 25 | 40 | 10 |
| 26 | 40 | 10 |
| 27 | 40 | 10 |
| 29 | 40 | 10 |
| 30 | 40 | 10 |
| 32 | 40 | 10 |
| 33 | 40 | 10 |
| 34 | 40 | 10 |
| 35 | 40 | 10 |
| 39 | 40 | 10 |
| 42 | 40 | 10 |
| 43 | 40 | 10 |
| 44 | 40 | 10 |

Test Example 5

Paddy field soil was filled in 1/5000 ares Wagner's pots, and the seeds of barnyardgrass were sowed in 1 to 2 cm depth in the soil. After creating the state of paddy field by flooding, rice seedlings in a 3-leaf stage were transplanted and cultivated in a greenhouse. After 5 days, a prescribed amount of the test compound formulated into emulsifiable concentrate or liquid formulation according to Formulation Example 2 or 5 was diluted with 10 mℓ of water and applied to the water surface, and the depth of water was made 4 cm. After treatment, the test plants were cultivated for 20 days in a greenhouse to examine the herbicidal activity. The results are shown in Table 8. In this test, water leakage corresponding to a water level of 3 cm/day was carried out for 2 days from the day subsequent to the treatment.

Table 8

| Compound No. | Dosage (g/are) | Herbicidal activity | |
|---|---|---|---|
| | | Rice | Barnyardgrass |
| 5 | 2.5<br>0.63 | 3<br>0 | 10<br>10 |
| 6 | 2.5<br>0.63 | 1<br>0 | 10<br>10 |
| 9 | 2.5<br>0.63 | 0<br>0 | 10<br>8 |
| 12 | 2.5<br>0.63 | 0<br>0 | 10<br>10 |
| 15 | 2.5<br>0.63 | 0<br>0 | 10<br>10 |
| 26 | 2.5<br>0.63 | 0<br>0 | 10<br>10 |
| 29 | 2.5<br>0.63 | 2<br>0 | 10<br>10 |
| 32 | 2.5<br>0.63 | 3<br>2 | 10<br>10 |
| 43 | 2.5<br>0.63 | 2<br>0 | 10<br>10 |
| A | 40<br>10 | 0<br>0 | 0<br>0 |
| B | 40<br>10 | 6<br>4 | 9<br>5 |
| C | 10<br>2.5 | 0<br>0 | 8<br>0 |
| D | 40<br>10 | 0<br>0 | 4<br>0 |

Test Example 6

Plow-field soil was filled in a vat (area: 33 x 23 $cm^2$, height: 11 cm) and the seeds of cotton, morning glories, common cocklebur, barnyardgrass and Johnsongrass were sowed and cultivated for 18 days. Thereafter, a prescribed amount of the test compound formulated into emulsifiable concentrate or liquid formulation according to Formulation Example 2 or 5 was diluted with a spreading agent-containing water of an amount corresponding to 5 liters/are, and uniformly applied to the entire foliage of the test plants over

the top by means of a small-sized sprayer. The state of growth of the weeds and crops at that time varied with the kind thereof, but they were in a cotyledonous stage and from 5 to 19 cm in height. Twenty days after treatment, the herbicidal activity was examined. The results are shown in Table 9.

These tests were carried out in a greenhouse through the entire period of test.

Table 9

| Compound No. | Dosage (g/are) | Herbicidal activity | | | | |
|---|---|---|---|---|---|---|
| | | Cotton | Morning-glories | Common cocklebur | Barnyard-grass | Johnson-grass |
| 7 | 20 | 0 | 8 | 7 | 8 | 7 |
| | 5 | 0 | 8 | - | 7 | - |
| 8 | 20 | 0 | 8 | 8 | 9 | 10 |
| | 5 | 0 | 7 | 8 | 8 | 9 |
| 10 | 20 | 0 | 8 | 8 | 7 | 8 |
| | 5 | 0 | 8 | 7 | - | - |
| 32 | 20 | 0 | 8 | 9 | 9 | 9 |
| | 5 | 0 | 8 | 8 | 9 | 9 |
| E | 20 | 2 | 8 | 5 | 5 | 5 |
| | 5 | 0 | 8 | 0 | 0 | 0 |

In addition to ingredients used in the Examples, Reference Example, Formulation Examples and Test Examples, other ingredients can be used in the Examples as set forth in the specification to obtain substantially the same results.

## Claims

### Claims for the following Contracting States : CH, DE, FR, GB, IT, LI, NL

**1.** A cinnoline derivative having the formula (I):

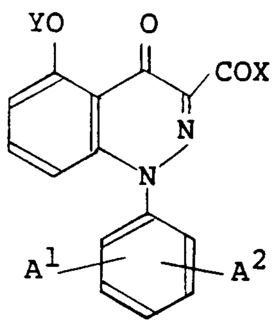

(I)

in which X is $-OH$, $-O^{-}M^{+}$, $-OR^1$ or

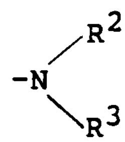

wherein $M^+$ is an alkali metal cation, an alkaline earth metal cation or

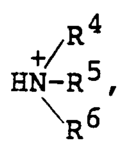

in which $R^4$, $R^5$ and $R^6$ are the same or different and each is a hydrogen atom, a $C_1-C_6$ alkyl group, a $C_3-C_4$ alkenyl group, a $C_3-C_4$ alkynyl group, a $C_3-C_8$ cycloalkyl group, a benzyl group or a phenyl group; $R^1$ is a $C_1-C_9$ alkyl group, a $C_3-C_6$ alkenyl group, a $C_3-C_4$ alkynyl group, a $C_1-C_3$ alkoxy-$(C_1-C_4)$ alkyl group, a $C_1-C_3$ haloalkyl group, a $C_3-C_8$ cycloalkyl group, a benzyl group or a phenyl group; and $R^2$ and $R^3$ are the same or different and each is a hydrogen atom, a $C_1-C_6$ alkyl group, a $C_3-C_4$ alkenyl group, a $C_3-C_4$ alkynyl group, a $C_3-C_8$ cycloalkyl group, a benzyl group in which at most two of hydrogen atoms at the $\alpha$-position thereof may be substituted by methyl group, a $C_2-C_3$ hydroxyalkyl group or a phenyl group in which at most three of hydrogen atoms thereof may be substituted by the same or different $C_1-C_2$ alkyl group or halogen atom;
Y is a $C_1-C_4$ haloalkyl group; $A^1$ and $A^2$ are the same or different and each is a hydrogen atom, a fluorine atom, a chlorine atom, a bromine atom, a trihalomethyl group, a $C_1-C_2$ alkylthio group or a $C_1-C_2$ haloalkoxy group.

**2.** The cinnoline derivative according to Claim 1, in which X is $-OH$, $-O^{-}M^{+}$ or $-OR^1$.

**3.** The cinnoline derivative according to Claim 2, in which $A^1$ is a fluorine atom, a chlorine atom, a bromine atom, a trifluoromethyl group, a difluoromethoxy group or a trifluoromethoxy group and $A^2$ is a hydrogen atom or a fluorine atom.

**4.** The cinnoline derivative according to Claim 3, in which Y is a $C_1-C_2$ polyfluoroalkyl group.

**5.** The cinnoline derivative according to Claim 4, in which Y is a difluoromethyl group.

**6.** The cinnoline derivative according to Claim 4, in which Y is a trifluoromethyl group.

**7.** The cinnoline derivative according to Claim 4, in which Y is a 2,2,2-trifluoroethyl group.

**8.** A process for preparing a cinnoline derivative having the formula (I-a):

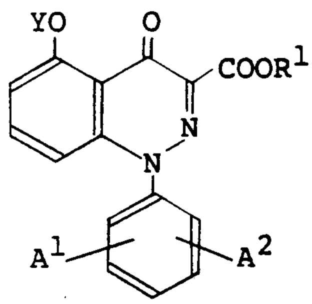

(I-a)

in which $R^1$ is a $C_1-C_9$ alkyl group, a $C_3-C_6$ alkenyl group, a $C_3-C_4$ alkynyl group, a $C_1-C_3$ alkoxy $(C_1-C_4)$ alkyl group, a $C_1-C_3$ haloalkyl group, a $C_3-C_8$ cycloalkyl group, a benzyl group or a phenyl group; Y is a $C_1-C_4$ haloalkyl group; $A^1$ and $A^2$ are the same or different and each is a hydrogen atom, a fluorine atom, a chlorine atom, a bromine atom, a trihalomethyl group, a $C_1-C_2$ alkylthio group or a $C_1-C_2$ haloalkoxy group which comprises subjecting a hydrazone derivative having the formula (II):

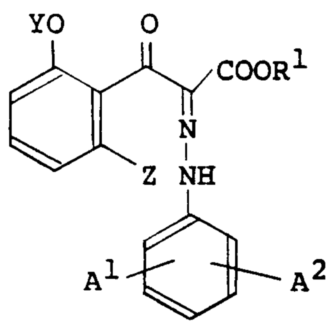

(II)

in which $R^1$, Y, $A^1$ and $A^2$ are as defined above; and Z is a fluorine atom, a chlorine atom or a bromine atom to ring closure with a dehydrohalogenating agent.

**9.** A process for preparing a cinnoline derivative having the formula (I-b):

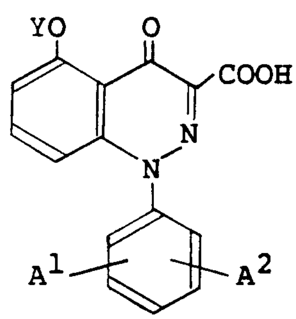

(I-b)

in which Y is a $C_1-C_4$ haloalkyl group; $A^1$ and $A^2$ are the same or different and each is a hydrogen atom, a fluorine atom, a chlorine atom, a bromine atom, a trihalomethyl group, a $C_1-C_2$ alkylthio group or a $C_1-C_2$ haloalkoxy group

which comprises hydrolyzing a cinnoline derivative having the formula (I – a):

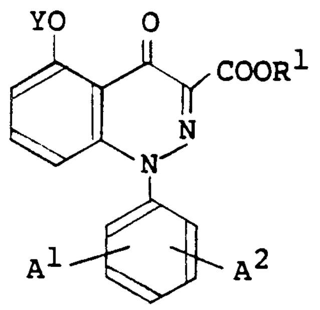

(I-a)

in which $R^1$ is a $C_1 - C_9$ alkyl group, a $C_3 - C_6$ alkenyl group, a $C_3 - C_4$ alkynyl group, a $C_1 - C_3$ alkoxy ($C_1 - C_4$) alkyl group, a $C_1 - C_3$ haloalkyl group, a $C_3 - C_8$ cycloalkyl group, a benzyl group or a phenyl group; and Y, $A^1$ and $A^2$ are as defined above.

**10.** A process for preparing a cinnoline derivative having the formula (I – c):

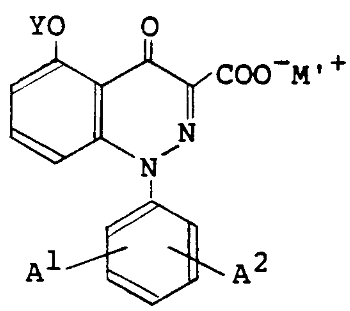

(I-c)

in which Y is a $C_1 - C_4$ haloalkyl group; $A^1$ and $A^2$ are the same or different and each is a hydrogen atom, a fluorine atom, a chlorine atom, a bromine atom, a trihalomethyl group, a $C_1 - C_2$ alkylthio group or a $C_1 - C_2$ haloalkoxy group; and $M'^{+}$ is an alkali metal cation or an alkaline earth metal cation
which comprises reacting a cinnoline derivative having the formula (I – b):

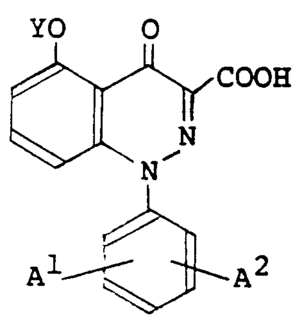

(I-b)

in which Y, $A^1$ and $A^2$ are as defined above
with a hydroxide having the formula (III):

$$M'^{+}OH^{-} \quad (III)$$

wherein $M'^{+}$ is as defined above.

**11.** A process for preparing a cinnoline derivative having the formula (I − d):

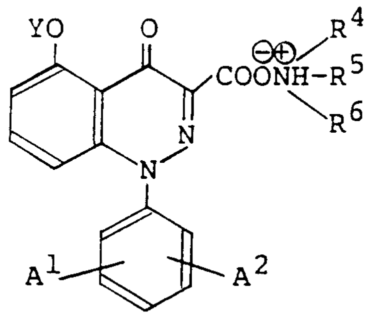

(I-d)

in which $R^4$, $R^5$ and $R^6$ are the same or different and each is a hydrogen atom, a $C_1-C_6$ alkyl group, a $C_3-C_4$ alkenyl group, a $C_3-C_4$ alkynyl group, a $C_3-C_8$ cycloalkyl group, a benzyl group or a phenyl group; Y is a $C_1-C_4$ haloalkyl group; $A^1$ and $A^2$ are the same or different and each is a hydrogen atom, a fluorine atom, a chlorine atom, a bromine atom, a trihalomethyl group, a $C_1-C_2$ alkylthio group or a $C_1-C_2$ haloalkoxy group
which comprises reacting a cinnoline derivative having the formula (I − b):

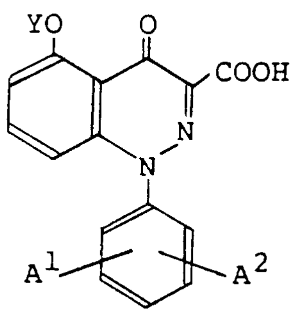

(I-b)

in which Y, $A^1$ and $A^2$ are as defined above
with an amine having the formula (IV):

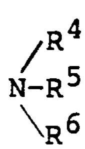

(IV)

in which $R^4$, $R^5$ and $R^6$ are as defined above.

**12.** A process for preparing a cinnoline derivative having the formula (I − e):

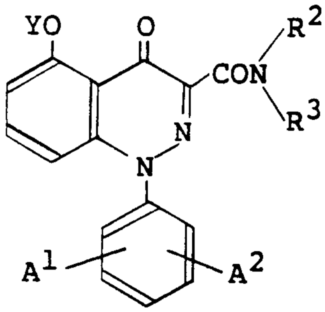

(I-e)

in which $R^2$ and $R^3$ are the same or different and each is a hydrogen atom, a $C_1-C_6$ alkyl group, a $C_3-C_4$ alkenyl group, a $C_3-C_4$ alkynyl group, a $C_3-C_8$ cycloalkyl group, a benzyl group wherein at most two of hydrogen atoms at the $\alpha$-position thereof may be substituted by methyl group, a $C_2-C_3$ hydroxyalkyl group or a phenyl group wherein at most three of hydrogen atoms thereof may be substituted by the same or different $C_1-C_2$ alkyl group or halogen atom; Y is a $C_1-C_4$ haloalkyl group; $A^1$ and $A^2$ are the same or different and each is a hydrogen atom, a fluorine atom, a chlorine atom, a bromine atom, a trihalomethyl group, a $C_1-C_2$ alkylthio group or a $C_1-C_2$ haloalkoxy group which comprises reacting a halide having the formula (V):

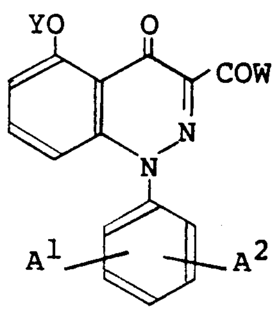

(V)

in which Y, $A^1$ and $A^2$ are as defined above; and W is a halogen atom with an amine having the formula (VI):

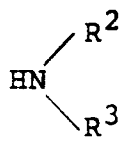

(VI)

in which $R^2$ and $R^3$ are as defined above.

**13.** A herbicidal composition comprising as an active ingredient a herbicidally effective amount of the compound according to Claim 1 and an inert carrier or dilutent.

**14.** A method for controlling undesired weeds, which comprises applying a herbicidally effective amount of the compound according to Claim 1 and an inert carrier or diluent to the area where undesired weeds grow or will grow.

**15.** Use of the compound according to Claim 1 as a herbicide.

**Claims for the following Contracting State : ES**

**1.** A process for preparing a cinnoline derivative having the formula (I-a):

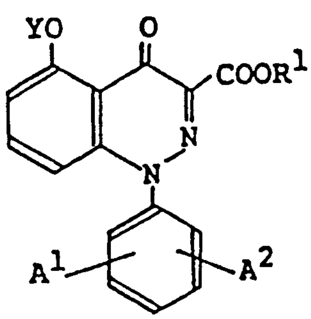

(I-a)

in which $R^1$ is a $C_1-C_9$ alkyl group, a $C_3-C_6$ alkenyl group, a $C_3-C_4$ alkynyl group, a $C_1-C_3$ alkoxy ($C_1-C_4$) alkyl group, a $C_1-C_3$ haloalkyl group, a $C_3-C_8$ cycloalkyl group, a benzyl group or a phenyl group; Y is a $C_1-C_4$ haloalkyl group; $A^1$ and $A^2$ are the same or different and each is a hydrogen atom, a fluorine atom, a chlorine atom, a bromine atom, a trihalomethyl group, a $C_1-C_2$ alkylthio group or a $C_1-C_2$ haloalkoxy group which comprises subjecting a hydrazone derivative having the formula (II):

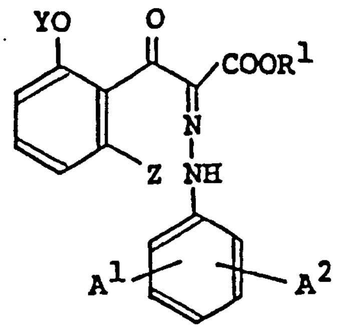

(II)

in which $R^1$, Y, $A^1$ and $A^2$ are as defined above; and Z is a fluorine atom, a chlorine atom or a bromine atom to ring closure with a dehydrohalogenating agent.

**2.** A process for preparing a cinnoline dervative having the formula (I-b):

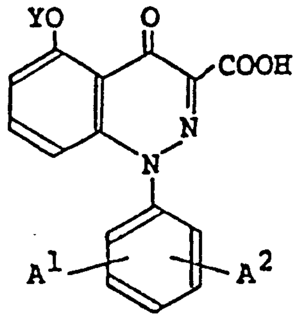

(I-b)

in which Y is a $C_1-C_4$ haloalkyl group; $A^1$ and $A^2$ are the same or different and each is a hydrogen atom, a fluorine atom, a chlorine atom, a bromine atom, a trihalomethyl group, a $C_1-C_2$ alkylthio group or a $C_1-C_2$ haloalkoxy group
which comprises hydrolyzing a cinnoline derivative having the formula (I-a):

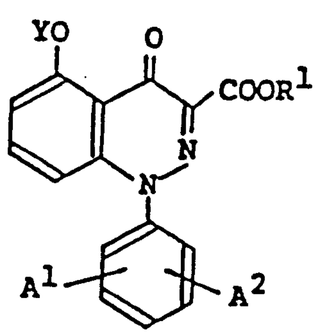

(I-a)

in which $R^1$ is a $C_1-C_9$ alkyl group, a $C_3-C_6$ alkenyl group, a $C_3-C_4$ alkynyl group, a $C_1-C_3$ alkoxy ($C_1-C_4$) alkyl group, a $C_1-C_3$ haloalkyl group, a $C_3-C_8$ cycloalkyl group, a benzyl group or a phenyl group; and Y, $A^1$ and $A^2$ are as defined above.

**3.** A process for preparing a cinnoline derivative having the formula (I – c):

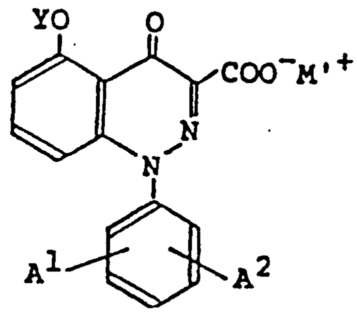

(I–c)

in which Y is a $C_1 - C_4$ haloalkyl group; $A^1$ and $A^2$ are the same or different and each is a hydrogen atom, a fluorine atom, a chlorine atom, a bromine atom, a trihalomethyl group, a $C_1 - C_2$ alkylthio group or a $C_1 - C_2$ haloalkoxy group; and $M'^{+}$ is an alkali metal cation or an alkaline earth metal cation which comprises reacting a cinnoline derivative having the formula (I – b):

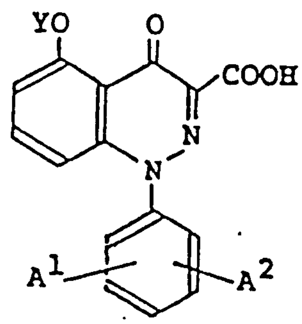

(I–b)

in which Y, $A^1$ and $A^2$ are as defined above with a hydroxide having the formula (III):

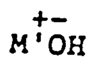

(III)

wherein $M'^{+}$ is as defined above.

**4.** A process for preparing a cinnoline derivative having the formula (I – d):

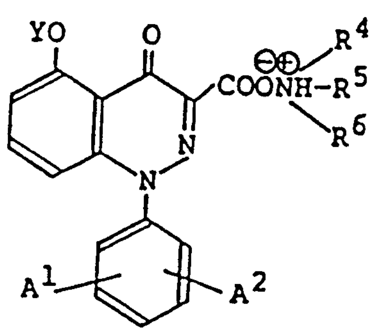

(I–d)

in which $R^4$, $R^5$ and $R^6$ are the same or different and each is a hydrogen atom, a $C_1 - C_6$ alkyl group, a $C_3 - C_4$ alkenyl group, a $C_3 - C_4$ alkynyl group, a $C_3 - C_8$ cycloalkyl group, a benzyl group or a phenyl group; Y is a $C_1 - C_4$ haloalkyl group; $A^1$ and $A^2$ are the same or different and each is a hydrogen atom, a fluorine atom, a chlorine atom, a bromine atom, a trihalomethyl group, a $C_1 - C_2$ alkylthio group or a

$C_1-C_2$ haloalkoxy group
which comprises reacting a cinnoline derivative having the formula (I-b)

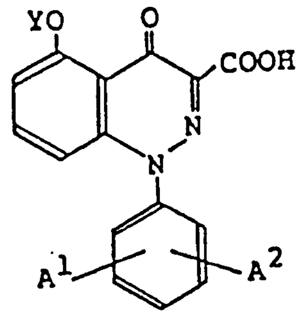

(I-b)

in which Y, $A^1$ and $A^2$ are as defined above with an amine having the formula (IV):

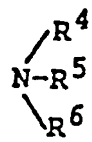

(IV)

in which $R^4$, $R^5$ and $R^6$ are as defined above.

**5.** A process for preparing a cinnoline derivative having the formula (I-e):

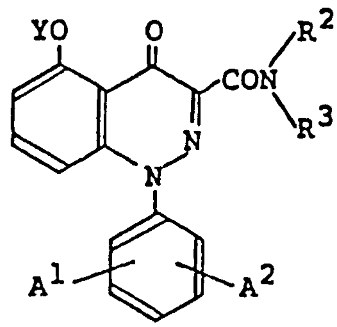

(I-e)

in which $R^2$ and $R^3$ are the same or different and each is a hydrogen atom, a $C_1-C_6$ alkyl group, a $C_3-C_4$ alkenyl group, a $C_3-C_4$ alkynyl group, a $C_3-C_8$ cycloalkyl group, a benzyl group wherein at most two of hydrogen atoms at the $\alpha$-position thereof may be substituted by methyl group, a $C_2-C_3$ hydroxyalkyl group or a phenyl group wherein at most three of hydrogen atoms thereof may be substituted by the same or different $C_1-C_2$ alkyl group or halogen atom; Y is a $C_1-C_4$ haloalkyl group; $A^1$ and $A^2$ are the same or different and each is a hydrogen atom, a fluorine atom, a chlorine atom, a bromine atom, a trihalomethyl group, a $C_1-C_2$ alkylthio group or a $C_1-C_2$ haloalkoxy group which comprises reacting a halide having the formula (V):

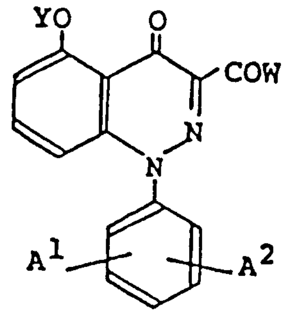

(V)

in which Y, $A^1$ and $A^2$ are as defined above; and W is a halogen atom with an amine having the formula (VI):

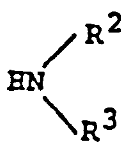

(VI)

in which $R^2$ and $R^3$ are as defined above.

6. A herbicidal composition comprising as an active ingredient a compound obtainable by a process according to Claims 1 to 5 in an amount of 0.1 to 90 % by weight and an inert carrier in an amount of 99.9 to 10 % by weight.

7. Process of use of the cinnoline derivatives (I-a) to (I-e) obtained in accordance with claims 1 to 5, for controlling undesired weeds, characterised in that it comprises applying a herbicidally effective amount of said compounds and a diluent or inert carrier to the area where undesired weeds grow or will grow.

**Patentansprüche**
**Patentansprüche für folgende Vertragsstaaten : CH, DE, FR, GB, IT, LI, NL**

1. Ein Cinnolinderivat der Formel (I):

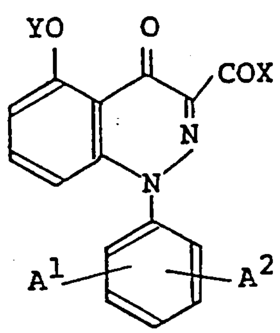

(I)

in der X $-OH$, $-O^-M^+$, $-OR^1$ oder

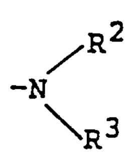

ist, worin $M^+$ ein Alkalimetallkation, ein Erdalkalimetallkation oder

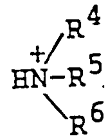

ist,
worin $R^4$, $R^5$ und $R^6$ gleich oder verschieden sind und jedes ein Wasserstoffatom, eine $C_1 - C_6$-Alkylgruppe, eine $C_3 - C_4$-Alkenylgruppe, eine $C_3 - C_4$-Alkinylgruppe, eine $C_3 - C_8$-Cylcoalkylgruppe, eine Benzylgruppe oder eine Phenylgruppe ist; $R^1$ eine $C_1 - C_9$-Alkylgruppe, eine $C_3 - C_6$-Alkenylgruppe, eine $C_3 - C_4$-Alkinylgruppe, eine $C_1 - C_3$-Alkoxy-$(C_1 - C_4)$-Alkylgruppe, eine $C_1 - C_3$-Halogenalkylgruppe, eine $C_3 - C_8$-Cycloalkylgruppe, eine Benzylgruppe oder eine Phenylgruppe ist; und $R^2$ und $R^3$ gleich oder verschieden sind und jedes ein Wasserstoffatom, eine $C_1 - C_6$-Alkylgruppe, eine $C_3 - C_4$-Alkenylgruppe, eine $C_3 - C_4$-Alkinylgruppe, eine $C_3 - C_8$-Cycloalkylgruppe, eine Benzylgruppe, in der höchstens zwei der Wasserstoffatome in der $\alpha$-Position durch eine Methylgruppe substituiert sein können, eine $C_2 - C_3$-Hydroxyalkylgruppe oder eine Phenylgruppe, in der höchstens drei der Wasserstoffatome durch gleiche oder verschiedene $C_1 - C_2$-Alkylgruppen oder Halogenatome substituiert sein können;
Y eine $C_1 - C_4$-Halogenalkylgruppe ist; $A^1$ und $A^2$ gleich oder verschieden sind und jedes ein Wasserstoffatom, ein Fluoratom, ein Chloratom, ein Bromatom, eine Trihalogenmethylgruppe, eine $C_1 - C_2$-Alkylthiogruppe oder eine $C_1 - C_2$-Halogenalkoxygruppe sind.

2. Cinnolinderivat nach Anspruch 1, bei dem X $-OH$, $-O^-M^+$ oder $-OR^1$ ist.

3. Cinnolinderivat nach Anspruch 2, bei dem $A^1$ ein Fluoratom, ein Chloratom, ein Bromatom, eine Trifluormethylgruppe, eine Difluormethoxygruppe oder eine Trifluormethoxygruppe ist und $A^2$ ein Wasserstoffatom oder ein Fluoratom ist.

4. Cinnolinderivat nach Anspruch 3, bei dem Y eine $C_1 - C_2$-Polyfluoralkylgruppe ist.

5. Cinnolinderivat nach Anspruch 4, bei dem Y eine Difluormethylgruppe ist.

6. Cinnolinderivat nach Anspruch 4, bei dem Y eine Trifluormethylgruppe ist.

7. Cinnolinderivat nach Anspruch 4, bei dem Y eine 2,2,2-Trifluorethylgruppe ist.

8. Verfahren zur Herstellung eines Cinnolinderivats der Formel (I-a):

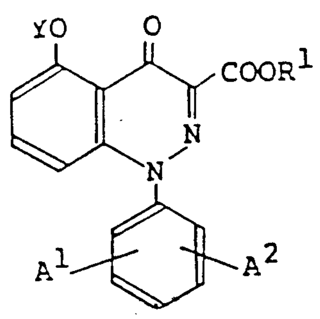

(I-a)

in der $R^1$ eine $C_1 - C_9$-Alkylgruppe, eine $C_3 - C_6$-Alkenylgruppe, eine $C_3 - C_4$-Alkynylgruppe, eine $C_1 - C_3$-Alkoxy-$(C_1 - C_4)$-Alkylgruppe, eine $C_1 - C_3$-Halogenalkylgruppe, eine $C_3 - C_8$-Cycloalkylgruppe, eine Benzylgruppe oder eine Phenylgruppe ist; Y eine $C_1 - C_4$-Halogenalkylgruppe ist; $A^1$ und $A^2$ gleich oder verschieden sind und jedes ein Wasserstoffatom, ein Fluoratom, ein Chloratom, ein Bromatom, eine Trihalogenmethylgruppe, eine $C_1 - C_2$-Alkylthiogruppe oder eine $C_1$

– $C_2$ – Halogenalkoxygruppe ist, welches die Cyclisierung eines Hydrazonderivates der Formel (II):

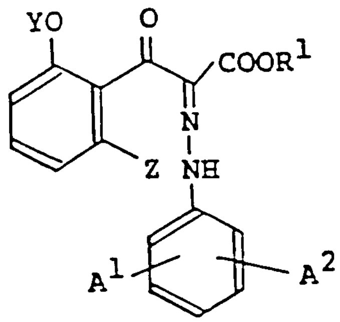

(II)

in der $R^1$, Y, $A^1$ und $A^2$ der obigen Definition entsprechen und Z ein Fluoratom, ein Chloratom oder ein Bromatom ist, mittels eines Dehydrohalogenierungsmittels umfaßt.

**9.** Verfahren zur Herstellung eines Cinnolinderivats der Formel (I – b):

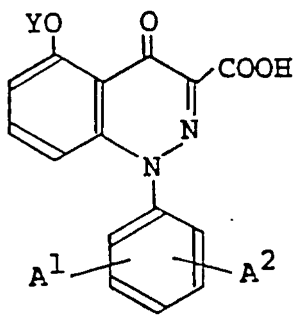

(I-b)

in der Y eine $C_1$ – $C_4$ – Halogenalkylgruppe ist; $A^1$ und $A^2$ gleich oder verschieden sind und jedes ein Wasserstoffatom, ein Fluoratom, ein Chloratom, ein Bromatom, eine Trihalogenmethylgruppe, eine $C_1$ – $C_2$ – Alkylthiogruppe oder eine $C_1$ – $C_2$ – Halogenalkoxygruppe ist, welches umfaßt die Hydrolyse eines Cinnolinderivats der Formel (I – a):

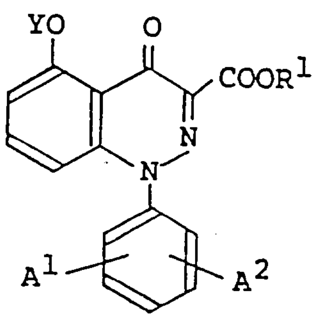

(I-a)

in der $R^1$ eine $C_1$ – $C_9$ – Alkylgruppe, eine $C_3$ – $C_6$ – Alkenylgruppe, eine $C_3$ – $C_4$ – Alkinylgruppe, eine $C_1$ – $C_3$ – Alkoxy – ($C_1$ – $C_4$) – Alkylgruppe, eine $C_1$ – $C_3$ – Halogenalkylgruppe, eine $C_3$ – $C_8$ – Cycloalkylgruppe, eine Benzylgruppe oder eine Phenylgruppe ist; und Y, $A^1$ und $A^2$ der obigen Definition entsprechen.

**10.** Verfahren zur Herstellung eines Cinnolinderivats der Formel (I – c):

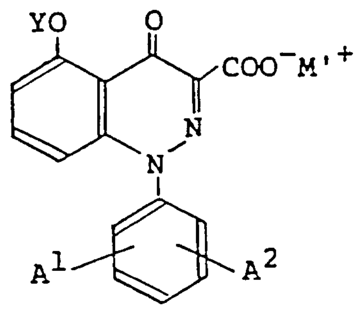

(I-c)

in der Y eine $C_1$ – $C_4$ – Halogenalkylgruppe ist; $A^1$ und $A^2$ gleich oder verschieden sind und jedes ein Wasserstoffatom, ein Fluoratom, ein Chloratom, ein Bromatom, eine Trihalogenmethylgruppe, eine $C_1$ – $C_2$ – Alkylthiogruppe oder eine $C_1$ – $C_2$ – Halogenalkoxygruppe ist; und $M'^+$ ein Alkalimetallkation oder Erdalkalimetallkation ist, welches umfaßt die Umsetzung eines Cinnolinderivats der Formel (I – b):

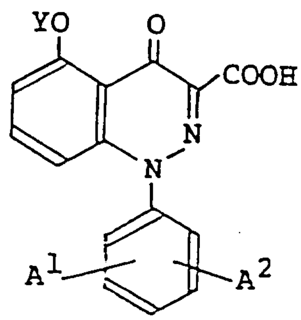

(I-b)

in der Y, $A^1$ und $A^2$ der obigen Definition entsprechen, mit einem Hydroxid der Formel (III):

$$\overset{+-}{M'OH} \qquad (III)$$

in der $M'^+$ der obigen Definition entspricht.

**11.** Verfahren zur Herstellung eines Cinnolinderivats der Formel (I – d):

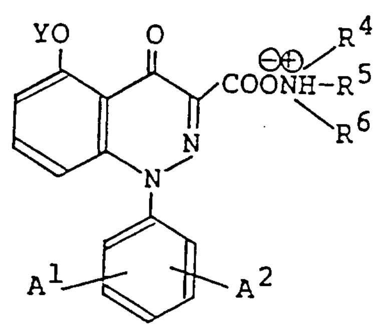

(I-d)

in der $R^4$, $R^5$ und $R^6$ gleich oder verschieden sind und jedes ein Wasserstoffatom, eine $C_1$ – $C_6$ – Alkylgruppe, eine $C_3$ – $C_4$ – Akenylgruppe, eine $C_3$ – $C_4$ – Alkinylgruppe, eine $C_3$ – $C_8$ – Cycloalkyl – gruppe, eine Benzylgruppe oder eine Phenylgruppe ist; Y eine $C_1$ – $C_4$ – Halogenalkylgruppe ist; $A^1$ und $A^2$ gleich oder verschieden sind und jedes ein Wasserstoffatom, ein Fluoratom, ein Chloratom, ein Bromatom, eine Trihalogenmethylgruppe, eine $C_1$ – $C_2$ – Alkylthiogruppe oder eine $C_1$ – $C_2$ –

Halogenalkoxygruppe ist, welches umfaßt die Umsetzung eines Cinnolinderivats der Formel (I-b):

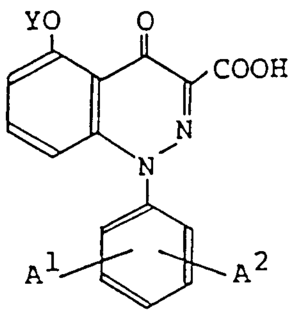

(I-b)

in der Y, $A^1$ und $A^2$ der obigen Definition entsprechen, mit einem Amin der Formel (IV):

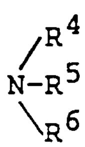

(IV)

in der $R^4$, $R^5$ und $R^6$ der obigen Definition entsprechen.

**12.** Verfahren zur Herstellung eines Cinnolinderivats der Formel (I-e):

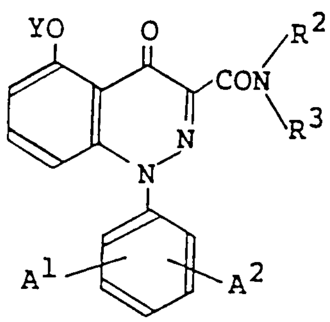

(I-e)

in der $R^2$ und $R^3$ gleich oder verschieden sind und jedes ein Wasserstoffatom, eine $C_1$ - $C_6$-Alkylgruppe, eine $C_3$ - $C_4$-Alkenylgruppe, eine $C_3$ - $C_4$-Alkinylgruppe, eine $C_3$ - $C_8$-Cycloalkylgruppe, eine Benzylgruppe, in der höchstens zwei der Wasserstoffatome in der $\alpha$-Position durch eine Methylgruppe substituiert sein können, eine $C_2$ - $C_3$-Hydroxyalkylgruppe oder eine Phenylgruppe, in der höchstens drei der Wasserstoffatome durch die gleiche oder verschiedene $C_1$ - $C_2$-Alkylgruppen oder Halogenatome substituiert sein können, ist; Y eine $C_1$ - $C_4$-Halogenalkylgruppe ist; $A^1$ und $A^2$ gleich oder verschieden sind und jedes ein Wasserstoffatom, ein Fluoratom, ein Chloratom, ein Bromatom, eine Trihalogenmethylgruppe, eine $C_1$ - $C_2$-Alkylthiogruppe oder eine $C_1$ - $C_2$-Halogenalkoxygruppe ist, welches umfaßt die Umsetzung eines Halogenids der Formel (V):

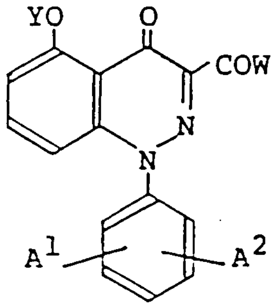

(V)

in der Y, $A^1$ und $A^2$ der obigen Definition entsprechen und W ein Halogenatom ist, mit einem Amin der Formel (VI):

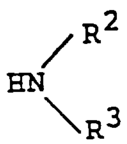

(VI)

in der $R^2$ und $R^3$ der obigen Definition entsprechen.

**13.** Eine herbizide Zusammensetzung enthaltend als Wirkstoff eine herbizid wirksame Menge der Verbindung nach Anspruch 1 und einen inerten Träger oder Verdünnungsmittel.

**14.** Verfahren zur Kontrolle unerwünschter Unkräuter, welches umfaßt die Anwendung einer herbizid wirksamen Menge der Verbindung nach Anspruch 1 und eines inerten Trägers oder Verdünnungsmittels auf die Fläche, auf der die unerwünschten Unkräuter wachsen oder wachsen werden.

**15.** Verwendung der Verbindung nach Anspruch 1 als Herbizid.

**Patentansprüche für folgenden Vertragsstaat : ES**

**1.** Ein Verfahren zur Herstellung eines Cinnolinderivats der Formel (I-a):

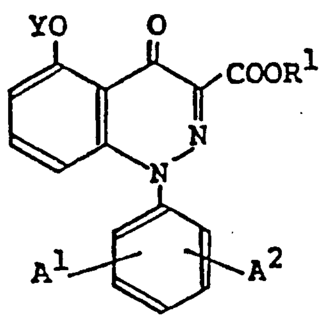

(I-a)

in der $R^1$ eine $C_1-C_9$-Alkylgruppe, eine $C_3-C_6$-Alkenylgruppe, eine $C_3-C_4$-Alkinylgruppe, eine $C_1-C_3$-Alkoxy-$(C_1-C_4)$-Alkylgruppe, eine $C_1-C_3$-Halogenalkylgruppe, eine $C_3-C_8$-Cycloalkylgruppe, eine Benzylgruppe oder eine Phenylgruppe ist; Y eine $C_1-C_4$-Halogenalkylgruppe ist; $A^1$ und $A^2$ gleich oder verschieden sind und jedes ein Wasserstoffatom, ein Fluoratom, ein Chloratom, ein Bromatom, eine Trihalogenmethylgruppe, eine $C_1-C_2$-Alkylthiogruppe oder eine $C_1-C_2$-Halogenalkoxygruppe ist, welches die Cyclisierung eines Hydrazonderivates der Formel (II):

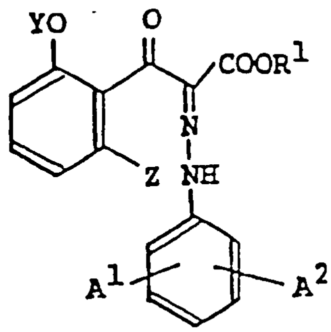

(II)

in der $R^1$, Y, $A^1$ und $A^2$ der obigen Definition entsprechen und Z ein Fluoratom, ein Chloratom oder ein Bromatom ist, mittels eines Dehydrohalogenierungsmittels umfaßt.

**2.** Verfahren zur Herstellung eines Cinnolinderivats der Formel (I-b):

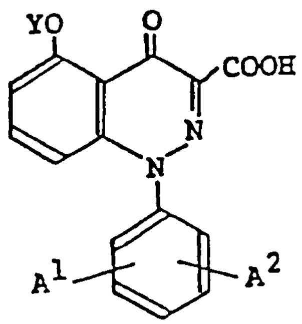

(I-b)

in der Y eine $C_1 - C_4$-Halogenalkylgruppe ist; $A^1$ und $A^2$ gleich oder verschieden sind und jedes ein Wasserstoffatom, ein Fluoratom, ein Chloratom, ein Bromatom, eine Trihalogenmethylgruppe, eine $C_1 - C_2$-Alkylthiogruppe oder eine $C_1 - C_2$-Halogenalkoxygruppe ist, welches umfaßt die Hydrolyse eines Cinnolinderivats der Formel (I-a):

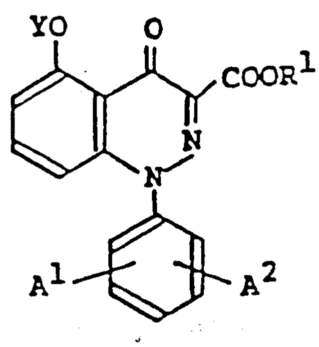

(I-a)

in der $R^1$ eine $C_1 - C_9$-Alkylgruppe, eine $C_3 - C_6$-Alkenylgruppe, eine $C_3 - C_4$-Alkinylgruppe, eine $C_1 - C_3$-Alkoxy-($C_1 - C_4$)-Alkylgruppe, eine $C_1 - C_3$-Halogenalkylgruppe, eine $C_3 - C_8$-Cycloalkylgruppe, eine Benzylgruppe oder eine Phenylgruppe ist; und Y, $A^1$ und $A^2$ der obigen Definition entsprechen.

**3.** Verfahren zur Herstellung eines Cinnolinderivats der Formel (I – c):

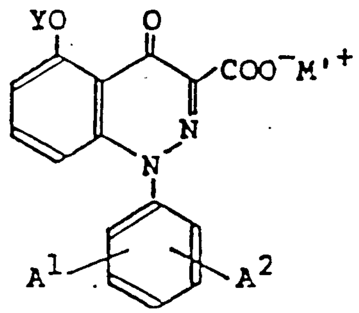

(I-c)

in der Y eine $C_1 - C_4$ – Halogenalkylgruppe ist; $A^1$ und $A^2$ gleich oder verschieden sind und jedes ein Wasserstoffatom, ein Fluoratom, ein Chloratom, ein Bromatom, eine Trihalogenmethylgruppe, eine $C_1 - C_2$ – Alkylthiogruppe oder eine $C_1 - C_2$ – Halogenalkoxygruppe ist; und $M'^{+}$ ein Alkalimetallkation oder Erdalkalimetallkation ist, welches umfaßt die Umsetzung eines Cinnolinderivats der Formel (I – b):

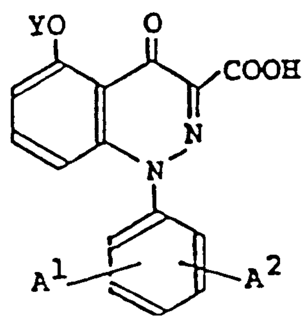

(I-b)

in der Y, $A^1$ und $A^2$ der obigen Definition entsprechen, mit einem Hydroxid der Formel (III):

$$\overset{+}{M'}\overset{-}{O}H$$

(III)

in der $M'^{+}$ der obigen Definition entspricht.

**4.** Verfahren zur Herstellung eines Cinnolinderivats der Formel (I – d):

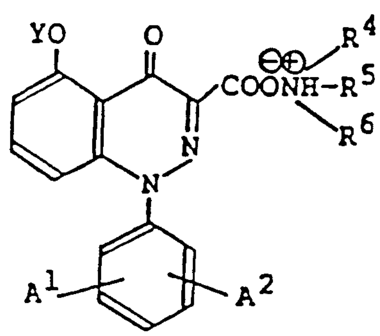

(I-d)

in der $R^4$, $R^5$ und $R^6$ gleich oder verschieden sind und jedes ein Wasserstoffatom, eine $C_1 - C_6$ – Alkylgruppe, eine $C_3 - C_4$ – Akenylgruppe, eine $C_3 - C_4$ – Alkinylgruppe, eine $C_3 - C_8$ – Cycloalkyl – gruppe, eine Benzylgruppe oder eine Phenylgruppe ist; Y eine $C_1 - C_4$ – Halogenalkylgruppe ist; $A^1$ und $A^2$ gleich oder verschieden sind und jedes ein Wasserstoffatom, ein Fluoratom, ein Chloratom, ein

Bromatom, eine Trihalogenmethylgruppe, eine $C_1 - C_2$-Alkylthiogruppe oder eine $C_1 - C_2$-Halogenalkoxygruppe ist, welches umfaßt die Umsetzung eines Cinnolinderivats der Formel (I-b):

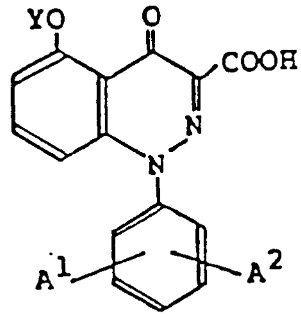

(I-b)

in der Y, $A^1$ und $A^2$ der obigen Definition entsprechen, mit einem Amin der Formel (IV):

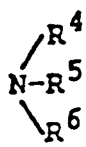

(IV)

in der $R^4$, $R^5$ und $R^6$ der obigen Definition entsprechen.

**5.** Verfahren zur Herstellung eines Cinnolinderivats der Formel (I-e):

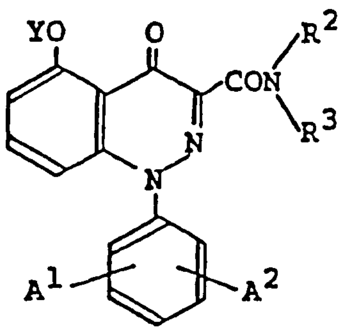

(I-e)

in der $R^2$ und $R^3$ gleich oder verschieden sind und jedes ein Wasserstoffatom, eine $C_1 - C_6$-Alkylgruppe, eine $C_3 - C_4$-Alkenylgruppe, eine $C_3 - C_4$-Alkinylgruppe, eine $C_3 - C_8$-Cycloalkylgruppe, eine Benzylgruppe, in der höchstens zwei der Wasserstoffatome in der $\alpha$-Position durch eine Methylgruppe substituiert sein können, eine $C_2 - C_3$-Hydroxyalkylgruppe oder eine Phenylgruppe, in der höchstens drei der Wasserstoffatome durch gleiche oder verschiedene $C_1 - C_2$-Alkylgruppen oder Halogenatome substituiert sein können, ist; Y eine $C_1 - C_4$-Halogenalkylgruppe ist; $A^1$ und $A^2$ gleich oder verschieden sind und jedes ein Wasserstoffatom, ein Fluoratom, ein Chloratom, ein Bromatom, eine Trihalogenmethylgruppe, eine $C_1 - C_2$-Alkylthiogruppe oder eine $C_1 - C_2$-Halogenalkoxygruppe ist, welches umfaßt die Umsetzung eines Halogenids der Formel (V):

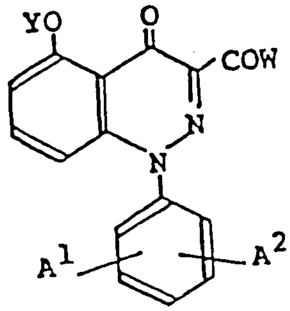

(V)

in der Y, $A^1$ und $A^2$ der obigen Definition entsprechen; und W ein Halogenatom ist, mit einem Amin der Formel (VI):

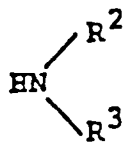

(VI)

in der $R^2$ und $R^3$ der obigen Definition entsprechen.

**6.** Herbizide Zusammensetzung enthaltend als Wirkstoff eine Verbindung erhältlich durch ein Verfahren nach den Ansprüchen 1 bis 5 in einer Menge von 0,1 bis 90 Gew. – % und einen inerten Träger in einer Menge von 99,9 bis 10 Gew. – %.

**7.** Verfahren zur Verwendung des Cinnolinderivats (I – a) bis (I – e) erhalten nach den Ansprüchen 1 bis 5 zur Kontrolle unerwünschter Unkräuter, dadurch gekennzeichnet, daß es umfaßt die Anwendung einer herbizid wirksamen Menge der genannten Verbindung und eines Verdünnungsmittels oder eines inerten Trägers auf die Fläche, auf der unerwünschte Unkräuter wachsen oder wachsen werden.

**Revendications**

**Revendications pour les Etats contractants suivants : CH, DE, FR, GB, IT, LI, NL**

**1.** Dérivé de cinnoline ayant la formule générale (I):

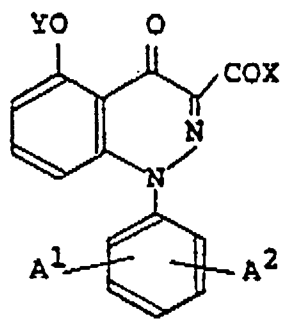

(I)

dans laquelle X représente un reste $-OH$, $-O^{-}M^{+}$, $-OR^1$ ou

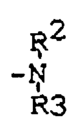

dans lequel $M^+$ représente un cation de métal alcalin, un cation de métal alcalino – terreux ou

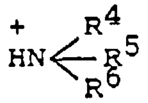 ,

dans lequel $R^4$, $R^5$ et $R^6$ sont identiques ou différents et chacun représente un atome d'hydrogène, un groupement alkyle en $C_1$ à $C_6$, un groupement alcényle en $C_3$ à $C_4$, un groupement alcynyle en $C_3$ à $C_4$, un groupement cycloalkyle en $C_3$ à $C_8$, un groupement benzyle ou un groupement phényle, $R^1$ représente un groupement alkyle en $C_1$ à $C_9$, un groupement alcényle en $C_3$ à $C_6$, un groupement alcynyle en $C_3$ à $C_4$, un groupement alkoxy (en $C_1$ à $C_3$)-alkyle (en $C_1$ à $C_4$), un groupement halogénoalkyle en $C_1$ à $C_3$, un groupement cycloalkyle en $C_3$ à $C_8$, un groupement benzyle ou un groupement phényle; et $R^2$ et $R^3$ sont identiques ou différents et chacun représente un atome d'hydrogène, un groupement alkyle en $C_1$ à $C_6$, un groupement alcényle en $C_3$ à $C_4$, un groupement alcynyle en $C_3$ à $C_4$, un groupement cycloalkyle en $C_3$ à $C_8$, un groupement benzyle dans lequel au plus deux de ses atomes d'hydrogène en position $\alpha$ peuvent être substitués par un groupement méthyle, un groupement hydroxyalkyle en $C_2$ à $C_3$ ou un groupement phényle dans lequel au plus trois de ses atomes d'hydrogène peuvent être substitués par un groupement alkyle en $C_1$ à $C_2$ identique ou différent ou par un atome d'halogène;
Y est un groupement halogénoalkyle en $C_1$ à $C_4$;
$A^1$ et $A^2$ sont identiques ou différents et chacun représente un atome d'hydrogène, un atome de fluor, un atome de chlore, un atome de brome, un groupement trihalogénométhyle, un groupement alkylthio en $C_1$ à $C_2$ ou un groupement halogénoalcoxy en $C_1$ à $C_2$.

**2.** Dérivé de cinnoline selon la revendication 1, dans lequel X représente un reste $-OH$, $-O^-M^+$ ou $-OR^1$.

**3.** Dérivé de cinnoline selon la revendication 2, dans lequel $A^1$ représente un atome de fluor, un atome de chlore, un atome de brome, un groupement trifluorométhyle, un groupement difluorométhoxy ou un groupement trifluorométhoxy et $A^2$ représente un atome d'hydrogène ou un atome de fluor.

**4.** Dérivé de cinnoline selon la revendication 3, dans lequel Y représente un groupement polyfluoroalkyle en $C_1$ à $C_2$.

**5.** Dérivé de cinnoline selon la revendication 4, dans lequel Y représente un groupement difluorométhyle.

**6.** Dérivé de cinnoline selon la revendication 4, dans lequel Y représente un groupement trifluorométhyle.

**7.** Dérivé de cinnoline selon la revendication 4, dans lequel Y est un groupement 2,2,2-trifluoroéthyle.

**8.** Procédé pour préparer un dérivé de cinnoline ayant la formule (I-a)

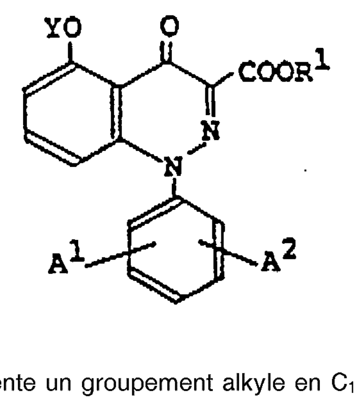

(I-a)

dans laquelle $R^1$ représente un groupement alkyle en $C_1$ à $C_9$, un groupement alcényle en $C_3$ à $C_6$, un groupement alcynyle en $C_3$ à $C_4$, un groupement alkoxy (en $C_1$ à $C_3$)-alkyle (en $C_1$ à $C_4$), un groupement halogénoalkyle en $C_1$ à $C_3$, un groupement cycloalkyle en $C_3$ à $C_8$, un groupement benzyle ou un groupement phényle; Y représente un groupement halogénoalkyle en $C_1$ à $C_4$; $A^1$ et $A^2$

sont identiques ou différents et chacun représente un atome d'hydrogène, un atome de fluor, un atome de chlore, un atome de brome, un groupement trihalogénométhyle, un groupement alkylthio en $C_1$ à $C_2$ ou un groupement halogénoalcoxy en $C_1$ à $C_2$ qui comprend l'étape consistant à soumettre un dérivé hydrazone ayant la formule (II):

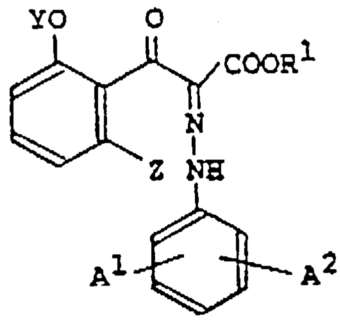

(II)

dans laquelle $R^1$, Y, $A^1$ et $A^2$ sont tels que définis ci-dessus; et Z représente un atome de fluor, un atome de chlore ou un atome de brome en vue d'une cyclisation avec un agent déhydrohalogénant.

**9.** Procédé pour préparer un dérivé de cinnoline ayant la formule (I-b):

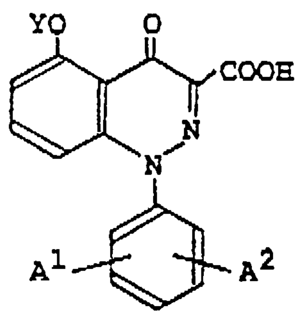

(I-b)

dans laquelle Y représente un groupement halogénoalkyle en $C_1$ à $C_4$; $A^1$ et $A^2$ sont identiques ou différents et chacun représente un atome d'hydrogène, un atome de fluor, un atome de chlore, un atome de brome, un groupement trihalogénométhyle, un groupement alkylthio en $C_1$ à $C_2$ ou un groupement halogénoalkoxy en $C_1$ à $C_2$
qui comprend l'étape consistant à hydrolyser un dérivé de cinnoline ayant la formule (I-a):

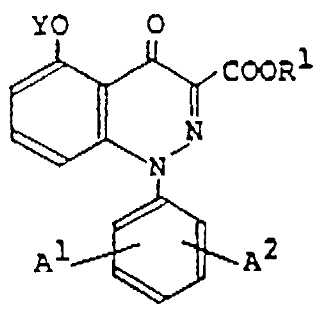

(I-a)

dans laquelle $R^1$ représente un groupement alkyle en $C_1$ à $C_9$, un groupement alcényle en $C_3$ à $C_6$, un groupement alcynyle en $C_3$ à $C_4$, un groupement alkoxy (en $C_1$ à $C_3$)-alkyle (en $C_1$ à $C_4$) un groupement halogénoalkyle en $C_1$ à $C_3$, un groupement cycloalkyle en $C_3$ à $C_8$, un groupement benzyle ou un groupement phényle; et Y, $A^1$ et $A^2$ sont tels que définis ci-dessus.

**10.** Procédé pour préparer un dérivé de cinnoline ayant la formule (I-c):

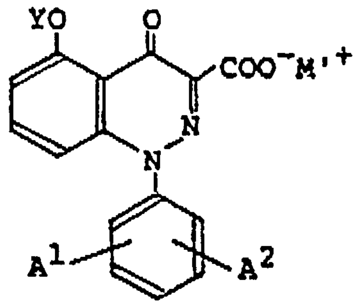

(I-c)

dans laquelle Y est un groupement halogénoalkyle en $C_1$ à $C_4$; $A^1$ et $A^2$ sont identiques ou différents et chacun représente un atome d'hydrogène, un atome de fluor, un atome de chlore, un atome de brome, un groupement trihalogénométhyle, un groupement alkylthio en $C_1$ à $C_2$ ou un groupement halogénoalkoxy en $C_1$ à $C_2$; et $M'^+$ représente un cation de métal alcalin ou un cation de métal alcalino-terreux

qui comprend l'étape consistant à faire réagir un dérivé de cinnoline ayant la formule (I-b):

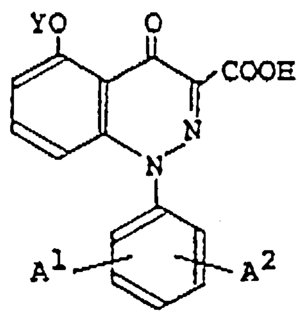

(I-b)

dans laquelle Y, $A^1$ et $A^2$ sont tels que définis ci-dessus avec un hydroxyde ayant la formule (III)

$M'^+OH^-$ (III)

dans laquelle $M'^+$ est tel que défini ci-dessus.

**11.** Procédé pour préparer un dérivé de cinnoline ayant la formule (I-d):

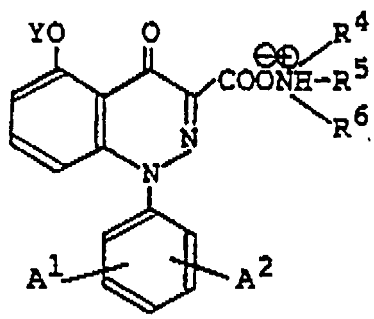

(I-d)

dans laquelle $R^4$, $R^5$ et $R^6$ sont identiques ou différents et chacun représente un atome d'hydrogène, un groupement alkyle en $C_1$ à $C_6$, un groupement alcényle en $C_3$ à $C_4$, un groupement alcynyle en $C_3$ à $C_4$, un groupement cycloalkyle en $C_3$ à $C_8$, un groupement benzyle ou un groupement phényle, Y représente un groupement halogénoalkyle; $A^1$ et $A^2$ sont identiques ou différents et chacun représente un atome d'hydrogène, un atome de fluor, un atome de chlore, un atome de brome, un groupement

trihalogénométhyle, un groupement alkylthio en $C_1$ à $C_2$ ou un groupement halogénoalkoxy en $C_1$ à $C_2$ qui comprend l'étape consistant à faire réagir un dérivé de cinnoline ayant la formule (I-b):

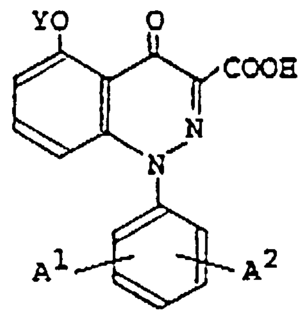

(I-b)

dans laquelle Y, $A^1$ et $A^2$ sont tels que définis ci-dessus avec une amine ayant la formule (IV):

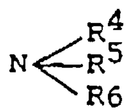

(IV)

dans laquelle $R^4$, $R^5$ et $R^6$ sont définis comme ci-dessus.

**12.** Procédé pour la préparation d'un dérivé de cinnoline ayant la formule (I-e):

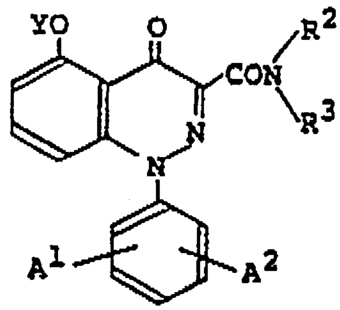

(I-e)

dans laquelle $R^2$ et $R^3$ sont identiques ou différents et chacun représente un atome d'hydrogène, un groupement alkyle en $C_1$ à $C_6$, un groupement alcényle en $C_3$ à $C_4$, un groupement alcynyle en $C_3$ à $C_4$, un groupement cycloalkyle en $C_3$ à $C_8O$, un groupement benzyle dans lequel au plus deux de ses atomes d'hydrogène en position $\alpha$ peuvent être substitués par un groupement méthyle, un groupement hydroxyalkyle en $C_2$ à $C_3$ ou un groupement phényle dans lequel au plus trois de ses atomes d'hydrogène peuvent être substitués par un groupement alkyle en $C_1$ à $C_2$ identique ou différent ou un atome d'halogène; Y représente un groupement halogénoalkyle en $C_1$ à $C_4$; $A^1$ et $A^2$ sont identiques ou différents et chacun représente un atome d'hydrogène, un atome de fluor, un atome de chlore, un atome de brome, un groupement trihalogénométhyle, un groupement alkylthio en $C_1$ à $C_2$ ou un groupement halogénoalkoxy en $C_1$ à $C_2$
qui comprend l'étape consistant à faire réagir un halogénure ayant la formule (V):

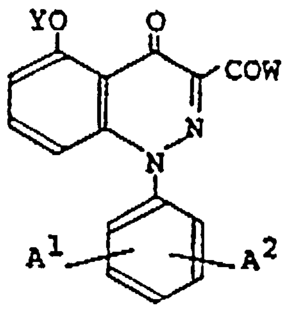

(V)

dans laquelle Y, $A^1$ et $A^2$ sont définis comme ci-dessus; et W représente un atome d'halogène avec une amine ayant la formule (VI)

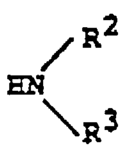

(VI)

dans laquelle $R^2$ et $R^3$ sont définis comme ci-dessus.

**13.** Composition herbicide comprenant comme principe actif une quantité efficace sur le plan herbicide du composé selon la revendication 1 et un support ou diluant inerte.

**14.** Procédé pour maîtriser les mauvaises herbes indésirées qui comprend l'étape consistant à appliquer une quantité efficace sur le plan herbicide du composé selon la revendication 1 et un support ou diluant inerte à la zone où des mauvaises herbes poussent ou pousseront.

**15.** Utilisation du composé selon la revendication 1 en tant qu'herbicide.

**Revendications pour l'Etat contractant suivant : ES**

**1.** Procédé pour préparer un dérivé de cinnoline ayant la formule (I-a)

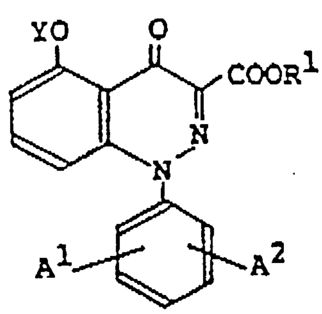

(I-a)

dans laquelle $R^1$ représente un groupement alkyle en $C_1$ à $C_9$, un groupement alcényle en $C_3$ à $C_6$, un groupement alcynyle en $C_3$ à $C_4$, un groupement alkoxy (en $C_1$ à $C_3$)-alkyle (en $C_1$ à $C_4$), un groupement halogénoalkyle en $C_1$ à $C_3$, un groupement cycloalkyle en $C_3$ à $C_8$, un groupement benzyle ou un groupement phényle; Y représente un groupement halogénoalkyle en $C_1$ à $C_4$; $A^1$ et $A^2$ sont identiques ou différents et chacun représente un atome d'hydrogène, un atome de fluor, un atome de chlore, un atome de brome, un groupement trihalogénométhyle, un groupement alkylthio en $C_1$ à $C_2$ ou un groupement halogénoalcoxy en $C_1$ à $C_2$ qui comprend l'étape consistant à soumettre un dérivé hydrazone ayant la formule (II):

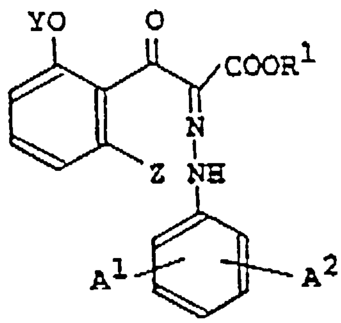

(II)

dans laquelle $R^1$ Y, $A^1$ et $A^2$ sont tels que définis ci-dessus; et
Z représente un atome de fluor, un atome de chlore ou un atome de brome en vue d'une cyclisation avec un agent déhydrohalogénant.

**2.** Procédé pour préparer un dérivé de cinnoline ayant la formule (I-b):

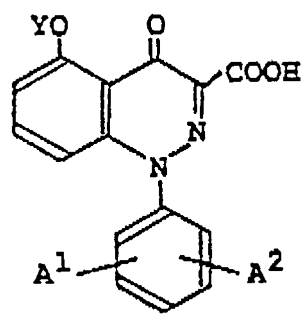

(I-b)

dans laquelle Y représente un groupement halogénoalkyle en $C_1$ à $C_4$; $A^1$ et $A^2$ sont identiques ou différents et chacun représente un atome d'hydrogène, un atome de fluor, un atome de chlore, un atome de brome, un groupement trihalogénométhyle, un groupement alkylthio en $C_1$ à $C_2$ ou un groupement halogénoalkoxy en $C_1$ à $C_2$
qui comprend l'étape consistant à hydrolyser un dérivé de cinnoline ayant la formule (I-a):

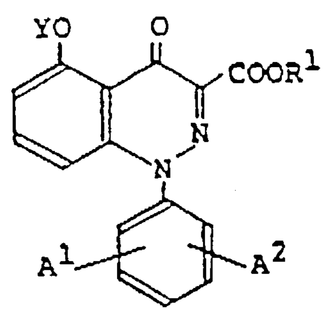

(I-a)

dans laquelle $R^1$ représente un groupement alkyle en $C_1$ à $C_9$, un groupement alcényle en $C_3$ à $C_6$, un groupement alcynyle en $C_3$ à $C_4$, un groupement alkoxy (en $C_1$ à $C_3$)-alkyle (en $C_1$ à $C_4$) un groupement halogénoalkyle en $C_1$ à $C_3$, un groupement cycloalkyle en $C_3$ à $C_8$, un groupement benzyle ou un groupement phényle; et Y, $A^1$ et $A^2$ sont tels que définis ci-dessus.

3. Procédé pour préparer un dérivé de cinnoline ayant la formule (I-c):

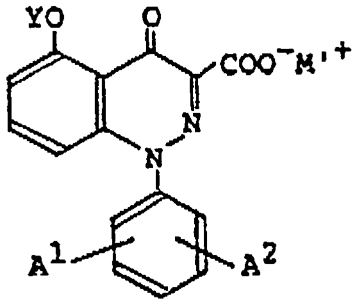

(I-c)

dans laquelle Y est un groupement halogénoalkyle en $C_1$ à $C_4$; $A^1$ et $A^2$ sont identiques ou différents et chacun représente un atome d'hydrogène, un atome de fluor, un atome de chlore, un atome de brome, un groupement trihalogénométhyle, un groupement alkylthio en $C_1$ à $C_2$ ou un groupement halogénoalkoxy en $C_1$ à $C_2$; et $M'^+$ représente un cation de métal alcalin ou un cation de métal alcalino-terreux
qui comprend l'étape consistant à faire réagir un dérivé de cinnoline ayant la formule (I-b):

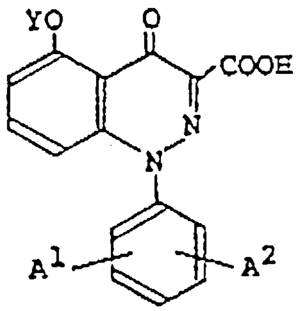

(I-b)

dans laquelle Y, $A^1$ et $A^2$ sont tels que définis ci-dessus avec un hydroxyde ayant la formule (III)

$M'^+OH^-$ (III)

dans laquelle $M'^+$ est tel que défini ci-dessus.

4. Procédé pour préparer un dérivé de cinnoline ayant la formule (I-d):

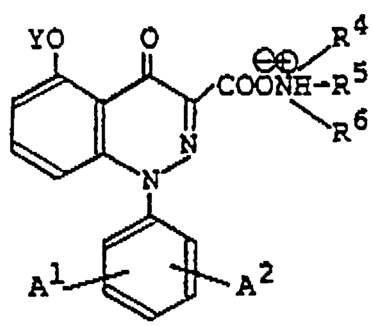

(I-d)

dans laquelle $R^4$, $R^5$ et $R^6$ sont identiques ou différents et chacun représente un atome d'hydrogène, un groupement alkyle en $C_1$ à $C_6$, un groupement alcényle en $C_3$ à $C_4$, un groupement alcynyle en $C_3$ à $C_4$, un groupement cycloalkyle en $C_3$ à $C_8$, un groupement benzyle ou un groupement phényle, Y représente un groupement halogénoalkyle; $A^1$ et $A^2$ sont identiques ou différents et chacun représente un atome d'hydrogène, un atome de fluor, un atome de chlore, un atome de brome, un groupement trihalogénométhyle, un groupement alkylthio en $C_1$ à $C_2$ ou un groupement halogénoalkoxy en $C_1$ à $C_2$

qui comprend l'étape consistant à faire réagir un dérivé de cinnoline ayant la formule (I-b):

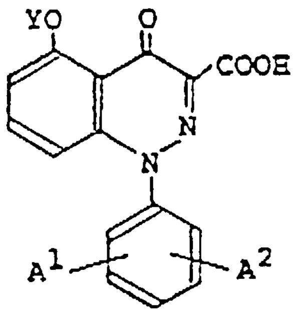

(I-b)

dans laquelle Y, $A^1$ et $A^2$ sont tels que définis ci-dessus avec une amine ayant la formule (IV):

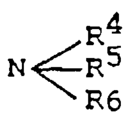

(IV)

dans laquelle $R^4$, $R^5$ et $R^6$ sont définis comme ci-dessus.

**5.** Procédé pour la préparation d'un dérivé de cinnoline ayant la formule (I-e):

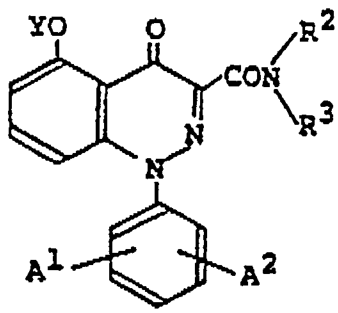

(I-e)

dans laquelle $R^2$ et $R^3$ sont identiques ou différents et chacun représente un atome d'hydrogène, un groupement alkyle en $C_1$ à $C_6$, un groupement alcényle en $C_3$ à $C_4$, un groupement alcynyle en $C_3$ à $C_4$, un groupement cycloalkyle en $C_3$ à $C_8$, un groupement benzyle dans lequel au plus deux de ses atomes d'hydrogène en position $\alpha$ peuvent être substitués par un groupement méthyle, un groupement hydroxyalkyle en $C_2$ à $C_3$ ou un groupement phényle dans lequel au plus trois de ses atomes d'hydrogène peuvent être substitués par un groupement alkyle en $C_1$ à $C_2$ identique ou différent ou un atome d'halogène; Y représente un groupement halogénoalkyle en $C_1$ à $C_4$; $A^1$ et $A^2$ sont identiques ou différents et chacun représente un atome d'hydrogène, un atome de fluor, un atome de chlore, un atome de brome, un groupement trihalogénométhyle, un groupement alkylthio en $C_1$ à $C_2$ ou un groupement halogénoalkoxy en $C_1$ à $C_2$
qui comprend l'étape consistant à faire réagir un halogénure ayant la formule (V):

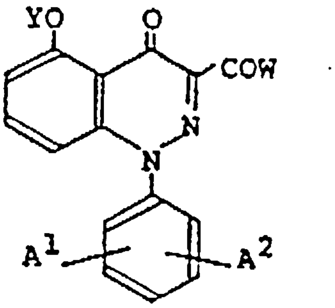

(V)

dans laquelle Y, $A^1$ et $A^2$ sont définis comme ci-dessus; et W représente un atome d'halogène avec une amine ayant la formule (VI)

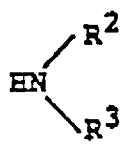

(VI)

dans laquelle $R^2$ et $R^3$ sont définis comme ci-dessus.

**6.** Composition herbicide comprenant comme principe actif un composé à obtenir par un procédé selon les revendications 1 à 5 dans une quantité de 0,1 à 90 % en poids et un support inerte dans une quantité de 99,9 à 10 % en poids.

**7.** Procédé pour utiliser le dérivé de cinnoline (I-a) à (I-e) obtenu selon les revendications 1 à 5 pour maitriser les mauvaises herbes indésirées, caracterisé en ce qu'il comprend l'application d'une quantité efficace dudit composé et d'un diluant on d'un support inerte à la zone où des mauvaises herbes poussent ou pousseront.